(19)

**Europäisches Patentamt**
**European Patent Office**
**Office européen des brevets**

(11) **EP 4 478 027 A1**

(12) **EUROPEAN PATENT APPLICATION**

(43) Date of publication:
**18.12.2024 Bulletin 2024/51**

(21) Application number: **23179532.9**

(22) Date of filing: **15.06.2023**

(51) International Patent Classification (IPC):
**G01N 15/14** (2024.01)    **G01N 15/10** (2024.01)

(52) Cooperative Patent Classification (CPC):
**G01N 15/1433; C12M 47/04; G01N 15/1425;**
**G01N 15/1429; G01N 15/1434;** C08F 299/028;
G01N 15/149; G01N 2015/1006; G01N 2015/1447;
G01N 2015/1452

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(71) Applicant: **Max-Planck-Gesellschaft zur**
**Förderung der**
**Wissenschaften e.V.**
**80539 München (DE)**

(72) Inventor: **The designation of the inventor has not**
**yet been filed**

(74) Representative: **Manitz Finsterwald**
**Patent- und Rechtsanwaltspartnerschaft mbB**
**Martin-Greif-Strasse 1**
**80336 München (DE)**

(54) **METHOD FOR IDENTIFYING AND SEPARATING A SPECIFIC COMPONENT OF INTEREST FROM A PLURALITY OF COMPONENTS, AND DEVICE**

(57)    The present invention relates to a method for identifying and separating a specific component of interest from a plurality of components, the method comprises the steps of: A) providing a dispersion comprising the plurality of components, optionally enclosed in compartments, and a photoresist, wherein among the plurality of components at least one portion shows a factor of interest; B) scanning of the dispersion for at least one factor of interest to identify said at least one portion among the plurality of components or optionally of the compartments and selecting those components or optionally compartments of interest and attributing a location to the components or optionally compartments of interest within the dispersion; C) applying a light to at least one part of the dispersion for curing said photoresist either attributed to the components of interest or not attributed to the components of interest; and D) separating a component from parts of the dispersion with cured photoresist from components from other parts of the dispersion with uncured photoresist. Further, the present invention relates to a device configured to carry out the method according to any one of the preceding claims.

*Fig.13*

**Description**

The present invention concerns a method for identifying and separating a specific component of interest from a plurality of components, and a device configured to carry out said method.

Sorting specific components, such as microparticles comprising natural cells, according to specific properties is a process required in a variety of biological studies and medical applications. Starting from a heterogeneous sample, specific components are isolated for further research, analysis and/or therapy, thereby circumventing contamination from other types of components. Aside from the extensive application of sorting methods in the fields of immunology and oncology, the interest of researchers from other disciplines, like tissue engineering and neuroscience, has increased over the last decades.

Current sorting methods can be categorized into two major categories, passive and active sorting. Passive sorting methods enrich components from samples utilizing a batch-type approach, whereby no analysis of individual components is required before making an active separation decision. Instead, components are brought into an environment where their behavior differs based on either physical properties or surface markers that are specific to only certain components. Non-limiting examples of passive sorting methods for components, such as natural cells, include Magnetic Activated Cell Sorting (MACS), Buoyancy Activated Cell Sorting (BACS), and cell sorting based on their different sedimentation rates. Non-limiting examples of active sorting methods for natural cells include Fluorescence Activated Cell Sorting (FACS). Each one of these methods is well known to the skilled person.

Another active sorting method-based approach relies on image analysis which can be divided into three subcategories, microfluidic flow, container-based flow and microarray platforms. Each one of these methods is well known to the skilled person [1]. Image-based sorting methods allow sorting based on morphological properties of the components. Considering natural cells, image-based sorting particularly allows sorting according to sub-cellular information, like the distribution of a protein within a cell, and does not always require a form of labelling.

To date, common sorting methods do not offer the possibility to observe individual components over an extended period of time, which would allow sorting based on time-lapse microscopy. Also, common sorting methods are often specialized for sorting based on only a single property and are inadequate in at least one of the following ways: low-throughput, complex sample preparation, requirement of extensive training or expensive equipment, low purity, low recovery, low viability, the need for fluorescence labelling or the inability to handle small-sized or large-sized samples.

The present invention solves the above problems of sorting particular compartments, such as natural cells, from a mixed sample. In particular, the present invention provides for a sorting method which is inexpensive, versatile and easy to use and handle - as compared to the common sorting techniques.

Specifically, the present invention relates to a method for identifying and separating a specific component of interest from a plurality of components, the method comprises the steps of: A) providing a dispersion comprising a plurality of components, optionally enclosed in compartments, and a photoresist, wherein among the plurality of components at least one portion shows a factor of interest; B) scanning of the dispersion for at least one factor of interest to identify said at least one portion among the plurality of components or optionally of the compartments and identifying those components or optionally compartments of interest and attributing a location to the components or optionally compartments of interest within the dispersion; C) applying a light to at least one part of the dispersion for curing said photoresist either attributed to the components of interest or not attributed to the components of interest; and D) separating a component from parts of the dispersion with cured photoresist from components from other parts of the dispersion with uncured photoresist.

As used herein, the term "specific component of interest" relates to a particular component which is either desired or undesired. In other words, the present invention enables the identification and separation, i.e. sorting, of desired components from a plurality of components comprising desired and undesired components. In addition or alternatively thereto, the present invention also enables the identification and separation of undesired components from a plurality of components comprising desired and undesired components.

As used herein, the term "component" refers to at least one component in a plurality of components (e.g., in a mixed sample) comprising said at least one component. It is therefore possible to sort a single component from a plurality of components, or to sort two or more components from a plurality of components. Two or more components may refer to at least two components of the same kind and/or of a different kind. For example, the plurality of components comprises component A and component B, wherein the present invention may sort two (A1, A2) or more (A1, A2, A3, and so on) components of kind A and/or two (B1, B2) or more (B1, B2, B3, and so on) components of kind B.

In step A) of the method in accordance with the present invention, a dispersion comprising a plurality of components and a photoresist is provided. Among the plurality of components at least one portion shows a factor of interest. Optionally, in step A) of the method in accordance with the present invention, a dispersion comprising a plurality of components enclosed in compartments and a photoresist is provided. Among the plurality of components enclosed in compartments at least one portion shows a factor of interest. The factor of interest may be a factor of interest of the component and/or a factor of interest of the compartment enclosing the component.

As used herein, the term "dispersion" refers to a dispersion (i.e., heterogeneous mixture of the component in the dispersion) or to a solution (i.e., homogeneous mixture of the component in the dispersion), preferably a dispersion. Said dispersion may be an aqueous or a non-aqueous dispersion comprising the photoresist and the plurality of components. As used herein, the term "aqueous dispersion" refers to a dispersion, wherein the continuous phase thereof is water. As used herein, the term "non-aqueous dispersion" refers to a dispersion, wherein the continuous phase thereof is an oil (e.g., fluorinated oil) or an organic solvent (e.g., dichloromethane, ether (such as dimethylether, diethylether, 2-Methoxypropane, tetrahydrofuran, or 1,4-dioxane), dimethyl sulfoxide, or a mixture thereof).

The photoresist may be soluble or insoluble in the continuous phase of the dispersion. The plurality of components is preferably soluble in the continuous phase of the dispersion but may also be insoluble in the continuous phase of the dispersion. In a preferred embodiment, the photoresist is soluble in a continuous phase, and the plurality of components is insoluble in said continuous phase. In another preferred embodiment, the photoresist is insoluble in a continuous phase, and the plurality of components is soluble in said continuous phase. In case a photoresist and a plurality of components have such different solubilities in a continuous phase, a separation of the photoresist from the plurality of components and vice versa may be improved. That way, a plurality of components having a higher purity may be obtained, in particular in case said plurality of components is intended for further investigation and/or application, e.g., analytical and/or therapeutic. It is possible that the component is insoluble in the continuous phase of the dispersion, e.g. in case the component is a particle, such as a natural cell, or an organic compound, such as a polymer. It is also possible that the plurality of components is soluble in the continuous phase of the dispersion, wherein in this case said dispersion refers to a solution. This may be the case, e.g., in case the component is a compound, such as DNA, RNA or a protein.

The photoresist may be incorporated in the continuous phase of the dispersion, e.g. by dissolving the photoresist in an aqueous phase in case the photoresist is water-soluble, or by dissolving the photoresist in a non-aqueous continuous phase in case the photoresist is not soluble in water but soluble in oil, for example. In case the component is enclosed in a compartment, the photoresist may be incorporated already at the time of forming the compartment, e.g. by microfluidic encapsulation, or at a later stage. The photoresist can also be injected into an existing compartment, by using an injection, which is preferably a pico-injector. Further, it is possible to incorporate the photoresist by diffusion into the compartment across the membrane itself or across pores in the membrane. It is also possible to use electroporation techniques for incorporating the photoresist into the compartment. Also, a combination of these techniques may be used.

In step B) of the method in accordance with the present invention, the dispersion is scanned for at least one factor of interest to identify said at least one portion among the plurality of components and identifying those components of interest and attributing a location to the components of interest within the dispersion. Optionally, in step B) of the method in accordance with the present invention, the dispersion is scanned for at least one factor of interest in order to identify said at least one portion among the plurality of compartments and selecting those compartments of interest and attributing a location to the compartments of interest within the dispersion. In a single scan in step B) each component and/or compartment may be examined for at least one factor of interest, such as for at least two different factors of interest. The selection in step B) may be performed depending on whether a factor of interest A, a factor of interest B, and/or factor of interest C is fulfilled or not fulfilled. For example, the selection in step B) may be performed on whether all factors of interest A, B, and C are fulfilled. In another example, the selection in step B) may be performed on whether at least one factor of interest is fulfilled (e.g., factor of interest A), and at least one factor of interest is not fulfilled (e.g., factors of interest B and C).

As used herein, the term "scanning of a dispersion" means the systematic examination of the dispersion with regard to at least one factor of interest in order to identify a component optionally encapsulated in a compartment, and attribute a location thereto. Depending on the size of the component optionally encapsulated in a compartment, a magnification may be required in step B). Said magnification may be performed by using any applicable technique, such as using a microscope. A magnification e.g. of 5x, 10x, 20x, 40x, 60x, 63x and 100x, respectively may be used for scanning a dispersion comprising compartments having an arithmetic mean diameter of between 20 $\mu$m to 200 $\mu$m.

The scanning step is essential for selecting desired components and/or components encapsulated in compartments inside the dispersion comprising desired and undesired components. In a preferred embodiment of the invention, the scanning rate at least partially depends on a shape detection. An efficient method of shape detection may therefore enhance the scanning rate, and eventually lead to a high throughput. The shape detection of components and/or compartments in a dispersion may be improved in case the components optionally encapsulated in compartments are arranged in a single layer. Superimposed components and/or components may impair the scanning process, and thus the selection process.

Furthermore, the scanning of the dispersion in step B) may be improved by using a uniform dispersion. That is, the components and/or compartments inside the dispersion may have a uniform morphology, size, arrangement to one another, distance to one another, and/or distribution in the dispersion.

The scanning in step B) is not limited to the scanning of a dispersion for identification of and attributing a location to specific components of interest - whether the components are encapsulated or not. It is also possible that a scanning may be utilized in order to select compartments which do not fulfill a factor of interest attributed to the compartments. For example, the scanning may be configured to identify and attribute a location to compartments which are empty, i.e., do not encapsulate a component. The scanning may also be configured to identify and attribute a location to compartments which have an undesired morphology, such as broken compartments, too small or too big compartments not fitting in the uniform morphology of the majority of compartments inside a dispersion.

In step C) of the method in accordance with the present invention, light is applied to at least one part of the dispersion for curing said photoresist either attributed to the components of interest or not attributed to the components of interest. In other words, the illumination may be performed on components optionally encapsulated in compartments which are of interest or those which are not of interest. These alternatives reflect the idea underlying the herein described invention that the separation is not limited to separating a desired fraction form an undesired fraction but also vice versa. The term fraction referring to a plurality or part of components optionally encapsulated in compartments.

By illuminating at least one part of a dispersion comprising a photoresist, said part may be cured. Alternative terms for "cured", as used herein, are "printed", "hardened", "polymerized" or "photopolymerized". The photoresist may polymerize such that said illuminated part thereof hardens. Due to the polymerization, the polymerized section may have a higher density than unpolymerized sections, for example when using PEGDA as photoresist. Therefore, hardened sections may have a different sedimentation speed than sections in which the photoresist which is not hardened.

Since a photoresist may be present in a compartment further encapsulating a component, the hardening of the photoresist inside said compartment may lead to said compartment having a different sedimentation speed than those compartments which comprises a photoresist which was not cured. The same applies to a photoresist which surrounds a component inside a dispersion, wherein the component may or may not be encapsulated in a compartment. Curing of said photoresist may then lead to a component embedded in a cured photoresist which may therefore have a different sedimentation speed than components which are embedded in a non-cured photoresist.

The sorting process described in the method according to the present invention is improved in case the illumination is performed in a uniform manner. That is, in order to facilitate the separation of one fraction from another fraction, it is preferred that the sedimentation rate within each fraction is at least substantially the same. In addition to a uniform arrangement of the components optionally encapsulated in a compartment, the illumination thereof should be performed in a substantially same manner, such as by using the same illumination technique with a light source comprising same illumination time, same illuminated area, same illumination intensity, same wavelength of light, and the like.

The mixture of cured and non-cured species (i.e., fractions) may be then separated from one another in step D) in accordance with the present invention. In step D) of the method in accordance with the present invention, a component is separated from parts of the dispersion with cured photoresist from components from other parts of the dispersion with uncured photoresist. Any applicable technique may be used, such as separation techniques based on different sedimentation speeds of the fractions. The term "sedimentation speed" refers to the rate at which a component and/or compartment sinks or settles within a heterogeneous mixture. The separation thereof is not limited to the sedimentation speed-based approach, but may also be based on crossflow filtration, centrifugation, filtration with a magnet, or size-cut off filters. Optionally, separation can be achieved by the fraction contained in cured photoresist sticking to a chamber and therefore remaining in the chamber in which the selection by photopolymerization has been carried out, while the fraction in uncured photoresist is flushed out of the chamber.

An optional step E) may be performed subsequent to step D), wherein step E) is directed to releasing the separated component encapsulated in a compartment from said compartment. Alternatively, an optional step E) may be performed prior to step D), wherein step E) is directed to releasing the component encapsulated in a compartment from said compartment. That way, the components as such may be obtained, e.g., for further analysis. Depending on the compartment used, different appropriate techniques may be used for the release. For example, if surfactant stabilized droplets are used as compartments, these may be broken by using an antistatic gun which may then result in the release of the components previously encapsulated. Alternatively, the compartments may be treated with a solution of a droplet-destabilizing surfactant, illumination of photocleavable surfactant, mechanical force-mediated fusion, or electric field-mediated fusion.

Shape and size of the compartment are not limited. The present invention can be realized on differently shaped and sized compartments. Nevertheless, in view of easiness of production, the compartment has an essentially round diameter. Further, in order to be able to encapsulate a component, the compartment has a size larger than that of the component, preferably an arithmetic mean diameter larger than that of a component to be encapsulated, or an arithmetic mean volume larger than that of a component to be encapsulated. More specifically, the diameter of the preferably round compartment is preferably in the range of 1 $\mu$m to 10.000 $\mu$m, preferably 3 $\mu$m to 1000 $\mu$m, more preferably 5 $\mu$m to 500 $\mu$m, and even more preferably 10 $\mu$m to 200 $\mu$m. As used herein, the term "diameter of the compartment" refers to an equivalent spherical diameter. In case of a round compartment, it is the outer diameter of the compartment. The diameter can be determined microscopically by measuring the diameter of a certain number of compartments, e.g. 50, 100 or 1000, and forming the arithmetic mean thereof. This may be realized by an automated image processing.

In a preferred embodiment, the compartment comprises a droplet, preferably a water-in-oil (W/O) droplet or an oil-in-water (O/W) droplet or a water-in-water (W/W) droplet, or a double emulsion droplet, such as a water-in-oil-in-water (W/O/W) droplet, an oil-in-water-in-oil (O/W/O) droplet, a water-in-water-in-oil (W/W/O) droplet, a water-in-water-in-water (W/W/W) droplet, an oil-in-oil-in-water (O/O/W) droplet; a vesicle, preferably a multilamellar vesicle, a unilamellar vesicle or a giant unilamellar vesicles (GUV); and/or a coacervate, in which the components are encapsulated. The droplet, as mentioned above, may be a surfactant stabilized droplet. The compartment is a spatially confined area having at least one wall which should allow for light propagation of the wavelength of interest, i.e. the wavelength for initiating photopolymerization in step c) of the method according to the present invention. Preferably, the compartment has a compartment wall defining the inside of the compartment and an outside thereof, wherein the compartment wall can have a surfactant-stabilized fluid interface with its environment. The at least one compartment wall may be a membrane, such as a lipid membrane, or a fluid shell. For example, a GUV has a lipid membrane, a droplet has a single wall, and a double emulsion droplet has two walls, i.e., an inner wall and an outer wall encapsulated said inner wall.

As used herein, the term "component" comprises a compound and/or a particle.

The "compound" may be a biological compound, an organic compound, an organometallic compound, and/or an inorganic compound. Non-limiting examples of the biological compound include peptides, biological macromolecules (e.g., proteins, and nucleic acids, such as RNA or DNA), molecular signatures (e.g., sets of genes, mRNA, transcripts, and proteins), biological toxins, signaling molecules (e.g., carbon monoxide (CO), ATP, p38 MAP kinases, HMGB1, nuclear DNA, cytokines comprising IL-1$\alpha$, and/or TGF-$\beta$, and hormones comprising insulin), and sub-cellular structures or organelles. Non-limiting examples of the organic compound includes polymers, such as LDPE (low-density polyethylene), HDPE (high-density polyethylene), PP (polypropylene), PUR (polyurethane), polyvinyl chloride (PVC), polystyrene (PS), nylon, and polytetrafluoroethylene (PTFE). Non-limiting examples of the organometallic compound include chlorophyll (chlorophyll a, chlorophyll b, chlorophyll $c_1$, chlorophyll $c_2$, chlorophyll d, and/or chlorophyll f).

The "particle" may be a particle having dimensions in the millimeter range, in the micrometer range (microparticle), and/or in the nanometer range (nanoparticle). As used herein, particles in the millimeter range may be organoids, such as cerebral organoid, gut organoid, lingual organoid, and thyroid organoid. As used herein, the microparticle and/or the nanoparticle may be a biological particle (i.e., naturally occurring particles, such as ferritin, magnetite, bacteria, viruses, spores, and pollen), an organic particle (e.g., pollen, and/or spores), an organometallic particle or an inorganic particle (e.g., ferritin, magnetite, and/or gold). The particle is preferably a biological particle in the nanometer range (biological nanoparticle). Non-limiting examples of the biological nanoparticle includes subcellular structures (e.g., magnetosomes), and extracellular structures (e.g., lipoproteins, exosomes, viruses). The particle is more preferably a biological particle in the micrometer range (biological microparticle). Non-limiting examples of the biological microparticle include natural cells (e.g., prokaryotic cells, archae and eukaryotic cells) and synthetic cells. The natural cell is preferably a eukaryotic cell, more preferably a cell from a cultured cell line, a stem cell, a cell from a model organism, like a mouse cell, a rat cell, a rabbit cell, a fish cell, even more preferably a primary human cell. In a particularly preferred embodiment, the natural cell is a mouse cancer cell, wherein the mouse cancer cell comprises sarcoma, such as J774A.1. In another particularly preferred embodiment, the natural cell is a human cancer cell comprising a circulating tumor cell (CTC). Non-limiting examples of a human cancer cell comprises A2780, A253, A2780cis, A549, CaCo-2, Calu-3, DAOY, DOV-434, DU145, H295R, HaCaT, HeLa, Hep G2, Jurkat, KPM-7, LNCaP, MCF-7, MDA-MB-468, NK-92, PC3, SaOS-2, SH-SY5Y, T-47D, THP-1, and U87). In another particularly preferred embodiment, the natural cell is a human primary cell, wherein the human primary cell comprises a hemocytoblast comprising a myeloid progenitor cell and a lymphoid progenitor cell. Non-limiting examples of a myeloid progenitor cell include a megakaryocyte (e.g., thrombocyte), erythrocyte, mast cell, and/or myeloblast (e.g., basophil, neutrophil, eosinophil, monocyte, and/or macrophage). Non-limiting examples of a lymphoid progenitor cell include a natural killer cell, small lymphocyte comprising a T cell, B cell and/or plasma cell. further example of a natural cell is a dendritic cell.

In a preferred embodiment, the component is a natural cell, synthetic cell, organelle, protein, nucleic acid, or inorganic particle. In a more preferred embodiment, the component is a natural cell.

In another preferred embodiment, the component of interest or optionally compartment of interest differs from the other components or optionally from the other compartments by an optical feature, such as a fluorescent moiety and/or the shape and/or behavior of the component. That is, a differentiation of a desired component or optionally compartment in step B) may be based on different optical qualities between the fractions in the dispersion.

For example, a first part of components may be attached to a fluorescent moiety, such as ROX or Fluorescein, while a second part of components is not attached to a fluorescent moiety (i.e., the second part is not fluorescent). In step B), a differentiation of the two parts may be achieved by screening for the first part of components, namely identification of the fluorescent components and attributing a location thereto. It is also possible that a first part of components may be attached to a first kind of fluorescent moiety, such as ROX, while a second part of components is attached to a second kind of fluorescent moiety, such as Fluorescein. In step B), a differentiation of the two parts may be achieved by screening for the first or the second part of components, namely identification of the first or the second kind of fluorescent light emitted by the first or the second part of components and attributing a location thereto.

It is also possible to differentiate the component and/or compartment of interest from the other components by means of morphology thereof, such as the size, shape, or structure thereof. Such a differentiation may be required in order to obtain a uniform dispersion. In other words, it may be possible to perform an initial "cleansing" of the dispersion by scanning for compartments and/or components which are not uniform as required or as one wishes, and then separating one fraction from another. For example, when lacking uniformity due to different sizes, separating a part of small compartments encapsulating components from another part of large compartments encapsulating components may be possible, or vice versa. After making the dispersion uniform as one wishes, it is possible to perform a further sorting method according to the present invention in order to screen for another optical feature. As already described above, a uniform dispersion may improve the shape detection of the compartment and/or the component.

It is also possible that a differentiation by means of morphology may be required in case natural cells are used as components and at least one part thereof is dead and therefore has a different size, shape and/or structure than living cells.

It is also possible that a differentiation by means of morphology may be required in case a cell division needs to be analyzed, and therefore those cells which divided or did not divide, for example inside a compartment, may be detected, screened for and separated from one another, as described herein.

The differentiation by means of a behavior of a component may comprise propagation of a compartment (such as cell division), decline of a compartment (such as any forms of cell death comprising apoptosis or necrosis), changes in a behavior based on a chemical reaction of at least one compartment (such as polymerization), changes in a behavior based on a physical reaction of at least one compartment (such as expansion or collapse), or shape or mobility of a compartment.

In another preferred embodiment, the photoresist is a negative photoresist and includes a monomer component and an initiator component. A negative photoresist is hardened by the exposure to light. It is also possible that the photoresist is a positive resist, which is solubilized by the exposure to light. Negative resists and positive resists are well known to the skilled person and the present invention is not limited to specific types of negative or positive resists.

In accordance with the present invention, the photoresist is preferably a negative resist, including a monomer component and an initiator component, wherein the monomer has more than one polymerizable group.

In a preferred embodiment, the photoresist is a biocompatible photoresist. The term "biocompatible photoresist" as used herein refers to a photoresist compatible with the component, such as a cell. The biocompatible photoresist may be non-toxic to the component (also referred to as "non-toxic photoresist") and/or may not permeate through a membrane of the component (also referred to as "non-permeable photoresist").

A non-toxic photoresist may be defined by a quality of not having toxic or injurious effects on the component, such as a cell. Such a biocompatible photoresist may even have no toxic or injurious effects on the component if it was penetrated by the biocompatible photoresist, such as by permeation of the biocompatible photoresist through the membrane of a cell. A non-limiting example of such a biocompatible photoresist is PEGDA. PEGDA may penetrate through a cell membrane but has no toxic or injurious effects on the cell - provided that the concentration of the PEGDA within the cell is low. At higher concentrations, PEGDA may not be referred to a "non-toxic photoresist" as used in the present invention. Further toxic photoresists as used herein are poly(ethyleneimine) or low molecular weight multi-arm polyethylenglycols. A non-limiting example of a "non-toxic photoresist" is a photoresist that does not form radicals. A further example of a non-toxic photoresist is a molecule that can undergo cycloaddition, preferably photoactivated cycloaddition. Molecules that can undergo photoactivated cycloadditions with themselves comprise coumarin, anthracene, cinnamic acid, and derivatives thereof (such as methylcoumarin, and pyrenyl cinnamic acid (PCA)). Molecules that can undergo photoactivated cycloadditions with another molecule comprise tetrazine and norbornene, tetrazine and trans-cyclooctene (TCO), tetrazole and cysteine (or other thiol containing molecules), cyclopropenone-caged dibenzocyclooctynes (Photo-DBCO) and azide, coumarin and thymine, and derivatives thereof.

Additionally, or alternatively to a "non-toxic photoresist", the term "biocompatible photoresist" as used herein may refer to a photoresist which substantially does not permeate through a membrane of a component, or does not permeate through a membrane of a component at all (non-permeable photoresist). In case the component is a cell, the biocompatible photoresist may not permeate through the cell membrane. A biocompatible photoresist is particularly preferred for a compound which includes a membrane, such as a natural cell, a synthetic cell, or an organelle. That way, it is possible to acquire a component which is substantially photoresist-free, or completely photoresist-free. The term "substantially photoresist-free component" refers to a component which includes a certain amount of photoresist, wherein said amount of photoresist does not have a toxic or injurious effect on the component, such as a cell. A substantially or completely photoresist-free component may have a higher purity, and may therefore be more qualified for further investigation and/or applications, such as therapeutic application by (re-)introducing said substantially or completely photoresist-free component into a subject. Non-limiting examples of such a "non-permeable photoresist" are photoresists that are conjugated to a cell membrane impermeable molecule, such as glucose and/or carboxylic acid, or to a cell membrane impermeable polymer, such as alginate, dextran, hyaluronic acid, and/or multi-arm polyethylene glycol derivatives (PEGs) optionally having a high molecular weight.

The concentration of the monomer component in the liquid is preferably in the range of 1 mg/ml to 500 mg/ml, preferably in the range of 1 mg/ml to 400 mg/ml, more preferably in the range of 1 mg/ml to 300 mg/ml, more preferably in the range of 1 mg/ml to 200 mg/ml, more preferably in the range of 1 mg/ml to 100 mg/ml, more preferably in the range of 1 mg/ml to 60 mg/ml, even more preferably in the range of 1 mg/ml to 50 mg/ml, even more preferably in the range of 5 mg/ml to 40 mg/ml, and even more preferably in the range of 10 mg/ml to 30 mg/ml.

Also, the concentration of the initiator component, preferably a photopolymerization initiator, can be varied over a broad range. The concentration of the initiator component in the liquid is preferably in the range of 0.5 mg/ml to 200 mg/ml, more preferably in the range of 1 mg/ml to 100 mg/ml, even more preferably in the range of 5 mg/ml to 50 mg/ml, even further more preferably in the range of 10 mg/ml to 30 mg/ml. With the concentration being in the range above, it is high enough to rapidly initiate the chemical reaction. For lower concentrations, printing a structure inside the compartment may not be possible. Further, the concentration is not too high. If the concentration of the initiator component is higher than indicated above, there is a risk that the chemical reaction is not only initiated at the focal point but also at other positions, where the light is not focused. If the concentration of the initiator component is higher than indicated above, there is additionally or alternatively a risk that the chemical reaction is occurring only between the initiator component and not with the monomer component, thereby inhibiting itself, and hence inhibiting an interaction with monomers and polymerization eventually.

A preferred example for a negative photoresist is one that includes a monomer component being capable of radical polymerization. Preferably the monomer component has more than one group derived from one of acrylic acid, acrylic acid esters, methacrylic acid, methacrylic acid esters, styrene, styrene derivatives, vinyl halides, e.g. vinyl chloride, vinyl esters, e.g. vinyl acetate, acrylonitrile, or allyl esters. A suitable and particularly preferred example is an acrylate based monomer component, such as polyethylene glycol) diacrylate (PEGDA) and a suitable photoinitiator, or a system including 1H,1H,2H,2H-tridecafluoro-n-octyl acrylate in combination with a cross-linker, such as bis(acryloyloxy)-2,2,3,3,4,4,5,5-octafluorohexane, and a suitable photoinitiator.

The type of photoinitiator is not particularly limited and the skilled person can select one that is known in the art. Examples for suitable photopolymerization initiators are acetophenone-based compounds, benzophenone-based compounds, benzoin-based compounds, thioxanthone-based compounds, anthraquinone-based compounds, azo compounds and others. Preferably, the photoinitiator is water soluble. A particularly preferred example for a photoinitiator is Lithium phenyl-2,4,6-trimethylbenzoylphosphinate (LAP), 2-Hydroxy-4'-(2-hydroxyethoxy)-2-methylpropiophenone (I2959), or 2,2-Dimethoxy-2-phenylacetophenone.

Further suitable photoresists are for example photoresists utilizing the polymerization of acidic polymers, such as alginate, alginate salts of monovalent ions, e.g. sodium alginate and so on, via photo-induced uncaging of caged divalent ions. Another suitable photoresist is one utilizing polymerization of proteins via photosensitizer, such as e.g. printing of protein BSA with photosensitizer rose. A further exemplary photoresist that may be used in the method of the present invention is a photoresist based on light-triggered click chemistry, which may be based on 1,3-dipolar cycloadditions, Diels-Alder reactions or thiol-ene addition. It is also possible that the photoresist is based on light triggered polymerization of polymerizable monomers, or monomer-initiator conjugates, such as DNA motifs with photocleavable spacer ends or light-induced heating causing caramelization of sugar.

In another preferred embodiment, the light used in step C) is an LED or a laser beam, in particular wherein the light is a focused light. In particular, the light may be a focused light, such as a focused laser beam, e.g., pulsed laser or a femtosecond pulsed laser, or a focused LED light. That way, selective targeting of the components and/or compartments having a quality of interest may be improved.

In another preferred embodiment, in step C), the light is configured to selectively illuminate the at least one part of the dispersion. It may be preferable to selectively illuminate a series of components and/or compartments and/or a limited number of components and/or compartments. Said limited number may be seven (7) or less components optionally encapsulated in compartments, or seven (7) or less component and/or compartments having a quality of interest or less, more preferably five (5) or less components optionally encapsulated in compartments, or five (5) or less component and/or compartments having a quality of interest or less, even more preferably three (3) or less components optionally encapsulated in compartments, or three (3) or less component and/or compartments having a quality of interest or less, even more preferably one (1) component optionally encapsulated in a compartment, or one (1) compartment having a quality of interest. This may be achieved by controlling the amount of light reaching said at least one part of the dispersion. For example, the use of an aperture may be performed for this, preferably an aperture together with a diaphragm placed in the optical path to limit the amount of light emitted by a system. An aperture (i.e., field aperture) as used in the technical field of optics, is well known to the skilled person. Preferably, an aperture size of f/2.8, f/4, f/5.6, f/8, f/11, f/16, or f/22 is used. When the aperture is not fully opened but narrow instead, e.g., with an aperture size of f/8, it is possible to minimize the illuminated are of the at least one part of the dispersion. That way, the field aperture may be adjusted such as to polymerize a single component and/or compartment having a quality of interest.

The wavelength of the light is not particularly limited and can be chosen by the skilled person based on the type of photoresist, in particular of the initiator, and the type of compartment or compartment wall, or component or component wall. According to a preferred embodiment, the wavelength of the light is around the wavelength of an absorption maximum of the initiator or around double the wavelength of the absorption maximum of the initiator. By applying light having around double the wavelength, two-photon absorption can be utilized allowing particularly high spatial resolution for preparing a targeted curing inside a compartment optionally encapsulating a component.

In another preferred embodiment, in step A) the dispersion is provided in a chamber having an array of sub-chambers, each being designed to take up one or more components. Such sub-chambers may be obtained by providing for walls inside a chamber. The walls may act as partition walls between the components optionally encapsulated in compartments in order to spatially separating said components from one another. This may facilitate the selective targeting of the components, in particular in case the light used for photopolymerization is not sufficiently focused, e.g., for a too small component and/or compartment. Non-limiting examples of such a chamber having sub-chambers is a commercially available microplate with 6, 12, 24, 48, 96, 384, 1536, 3456 or 9600 wells or custom-made microfluidic devices.

In another preferred embodiment, the chamber is designed to have only one layer of components, optionally enclosed in compartments, and/or wherein the concentration of components, optionally enclosed in compartments, in step A) is adjusted so that a single layer of components, optionally enclosed in compartments, is formed within the chamber. As already mentioned above, providing for a single layer of compartments and/or components in a chamber improves the shape detection of the compartments and/or components. A single layer may be required in case the scanning of the dispersion is performed form one angle only onto the dispersion, e.g., using a microscope and scanning the dispersion from above which is known for common microscopes. However, in case scanning may be performed from at least two different angles towards the dispersion, it may also be possible to effectively scan a dispersion having two layers of components and/or compartments.

Provision of a single layer of components and/or compartments may be facilitated by reducing the amount of air inside the chamber comprising the dispersion. Under optimal conditions, the chamber contains the dispersion, thereby excluding air (or any other gas present in the environmental surrounding during the experiment).

In another preferred embodiment, the method further comprises step B0) prior to step B), wherein B0) is scanning of the chamber to determine a suitable depth of focus for a camera observing the chamber. That way, repeated auto focusing for each tile which can be time-consuming when using a microscope may be avoided. In step B0), the microscope may first determine a height map for the surface area in which all the components optionally encapsulated in compartments are embedded (top of the chamber when encapsulated in compartments, bottom otherwise). That way, auto focusing may be performed at discrete positions only so that the focus height in between may be interpolated.

In another preferred embodiment, the method in accordance with the present invention is carried out by utilizing a microscope having a light source suitable for polymerizing the photoresist. In particular, steps B) and C) are carried out by utilizing a microscope having a light source suitable for polymerizing the photoresist. The light source may be chosen as one which provides for a light as described above. A non-limiting example of a microscope having a light source suitable for polymerizing the photoresist is a conventional microscope, an epifluorescence microscope, a confocal microscope, a multi-photon microscope or a superresolution microscope equipped with suitable light sources, camera and objective. It is particularly preferred that a fluroescence microscope with a motorized stage is used.

In another preferred embodiment, the method is carried out by utilizing a microscope having a camera, and wherein in step B) an image is taken by the camera and analyzed, in particular by a computer, for at least one factor of interest. Any suitable camera compatible with a microscope and with a sorting experiment may be used. Using the computer and a motorized stage may significantly increase the scanning rate in step B). The camera may take numerous images of the dispersion and the images may be send to a computer to analyse each image for a factor of interest.

In another preferred embodiment, the method according to the present invention is carried out by utilizing a microscope providing structured illumination, preferably wherein the microscope has a digital micromirror device, to allow curing of the photoresist in step C) at several positions in parallel. A digital-micromirror-device (DMD) uses hundreds of thousands of micrometer-sized mirrors to project light and can be used for structured illumination of a sample. The mirrors are arranged in a two-dimensional array and illuminated by a light source. By changing its tilt angle, each mirror individually can be set to an on- or off-state, to reflect light onto a target area or into a heatsink, respectively. Thereby, every mirror determines the illumination of one pixel making up the pattern that is projected onto the target area. That way, a DMD attached to a microscope can be used to enable simultaneous polymerization at numerous spots. Using a DMD may therefore provide for a higher throughput in the screening step of the herein described method.

In another preferred embodiment, the method is in part a computer-implemented method. In particular, the computer-implemented method may include in optional step B0) the scanning of the chamber being configured to determine a depth of focus for taking images of the chamber. Further, the computer-implemented method may include in step B) the acquisition of an image and analysis of the image for at least one factor of interest and attributing a location to the components of interest within the dispersion. Even further, the computer-implemented method may include in step C) controlling a light source to selectively apply light to the photoresist at a portion desired to cure said photoresist. The scanning performed in step B0) and/or B), and/or the curing step C) of the herein described method may hence be entirely performed by using a computer.

For example, the underlying software configured to control the automated scanning process in step B) may be divided into two parallel processes. The first process may be a macro, i.e., a script that is used to automate control of the microscope and determine the order of steps during the scanning process. The second process may be a separate piece of software responsible for the required data analysis. Said parallel processes may further be applicable mutatis mutandis with regard to step B0) and/or step C).

It is preferred that step B0), step B) and/or step C) is carried out automatically, in particular based on machine learning driven image analysis. This may be performed by using artificial intelligence, such as convolutional neural network (CNN).

Furthermore, it is preferred that in step B) the image acquisition is controlled by the computer analysing the image, adjusting the focus and/or coordinates of the image taken.

In another preferred embodiment, in step A) components are provided in an amount of at least $10^2$ cells per total volume of the dispersion, preferably in an amount lying in a range of from $10^5$ to $10^7$ cells per total volume of the dispersion. It may therefore be possible to screen for small- to medium-sized sample concentrations, such as $10^2$ to $10^4$ components per $5\mu L$ dispersion. This may be particularly advantageous in cases where the sample size and the concentration thereof is limited.

Another aspect of the present invention is directed to a device configured to carry out the method in accordance with the present invention. It is preferred that the device comprises a microscope, a camera, a DMD, a light source, and/or a combination thereof.

All details explained above or in the appended claims with regard to the method for identifying and separating a specific component of interest from a plurality of components apply mutatis mutandis also to the method being carried out by said device.

Next, the present invention will be described by reference to the drawings and examples. In the drawings, the following is shown:

Fig. 1: Schematic illustration of an embodiment, wherein the continuous phase of the dispersion (herein: aqueous phase) comprises a photoresist;

Fig. 2: Schematic illustration of an embodiment using water-in-oil-droplets encapsulating a plurality of components and a photoresist;

Fig. 3: Schematic illustration of an embodiment using water-in-oil-droplets encapsulating a plurality of components, and the continuous oil phase containing a photoresist inside a selection chamber;

Fig. 4: Schematic illustration of an embodiment using double emulsions being W/O/W droplets encapsulating a plurality of components;

Fig. 5a-b: Microscope images for assessment of the printing behavior of a water-soluble photoresist inside and "outside" of W/O droplets as described in Example 1;

Fig. 6a-c: Microscope images demonstrating an imprinting of W/O droplets with patterns as described in Example 2;

Fig. 7a-c: Microscope images demonstrating a precise imprinting of single W/O droplets as described in Example 3;

Fig. 8a-c: Determining the detection accuracy of W/O droplets using circle detection;

Fig. 9a-b: Microscope images demonstrating the effect of a condenser illumination on the circle detection;

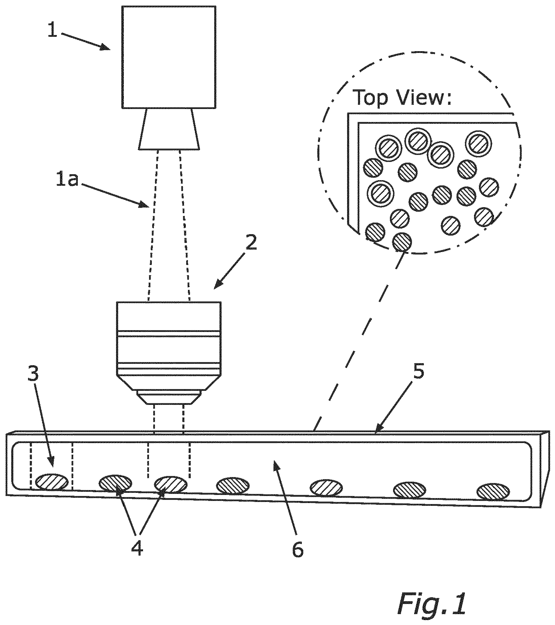

Fig. 1 0a-c: Illustration of different chamber prototypes;

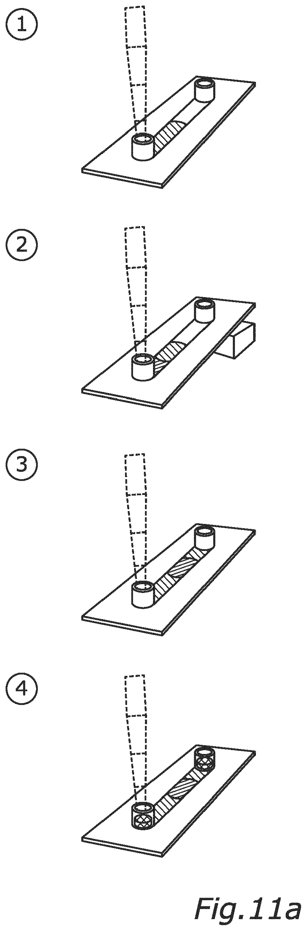

Fig. 11a: Workflow illustrating step A) as described in Example 7;

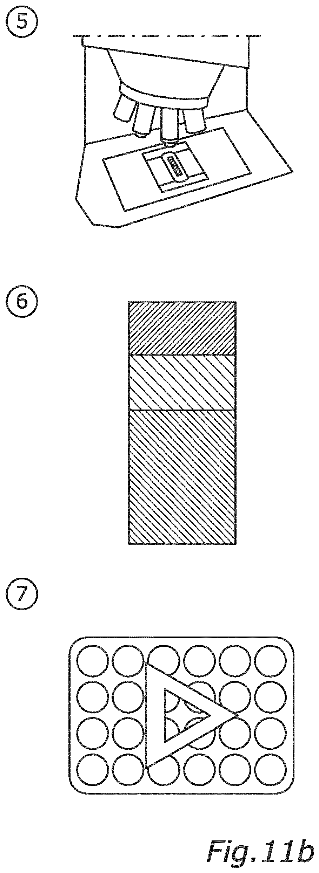

Fig. 11b: Workflow illustrating steps B) and C) as described in Example 7;

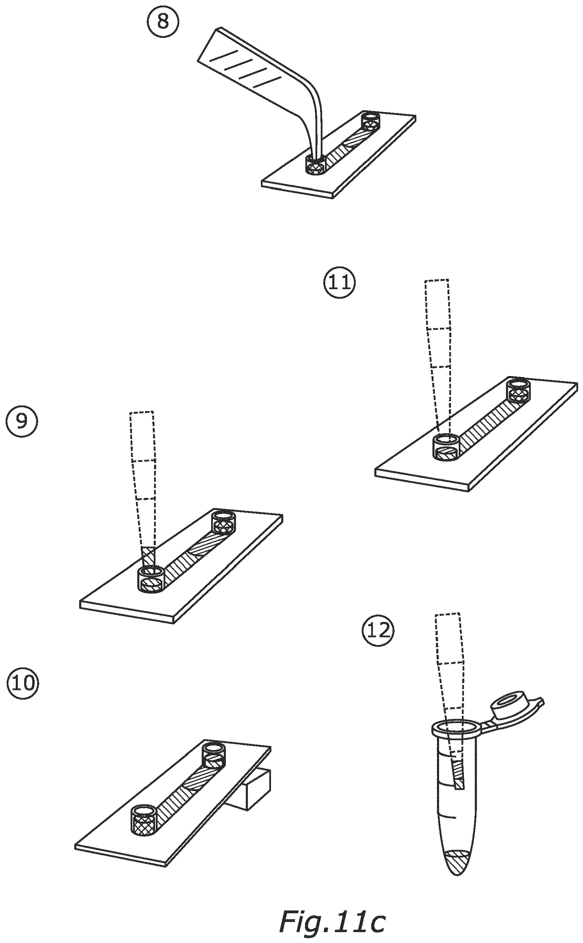

Fig. 11c: Workflow illustrating step D) as described in Example 7;

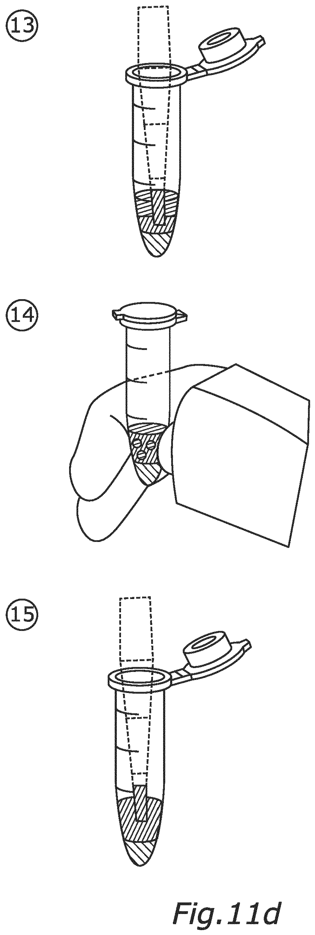

Fig. 11d: Workflow illustrating cell release as described in Example 7;

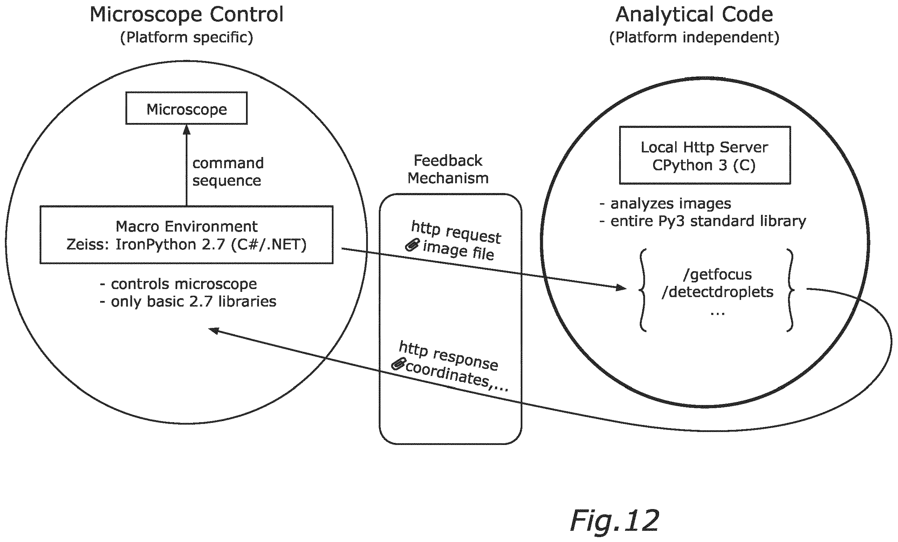

Fig. 12: Visualization of an example software architecture using a Zeiss microscope and macro scripting language IronPython 2.7 connected with separate image analysis scripts in CPython 3 environment used for the selection step in step C) as described in Example 7;

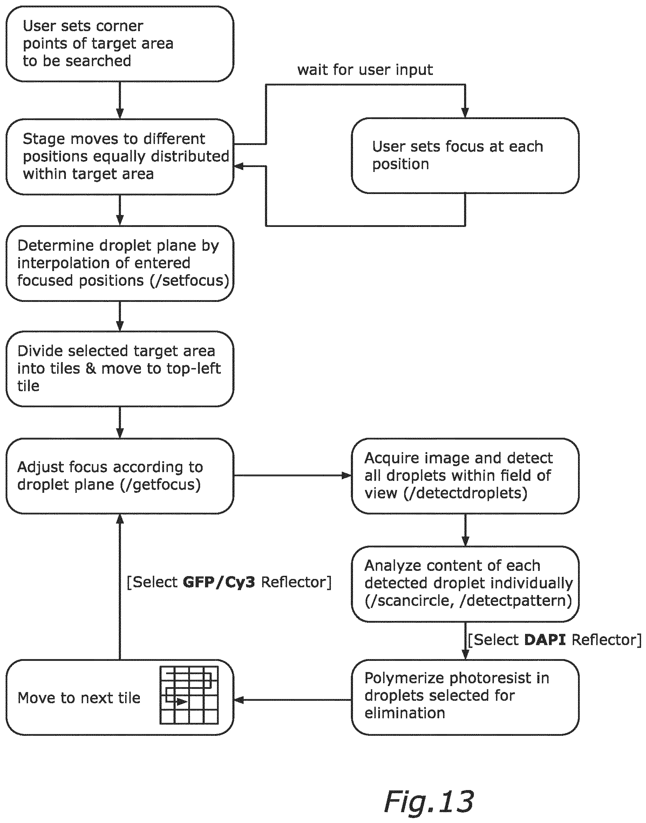

Fig. 13: Visualization of an example flow chart of a macro code executed in the selection in step C) as described in Example 7;

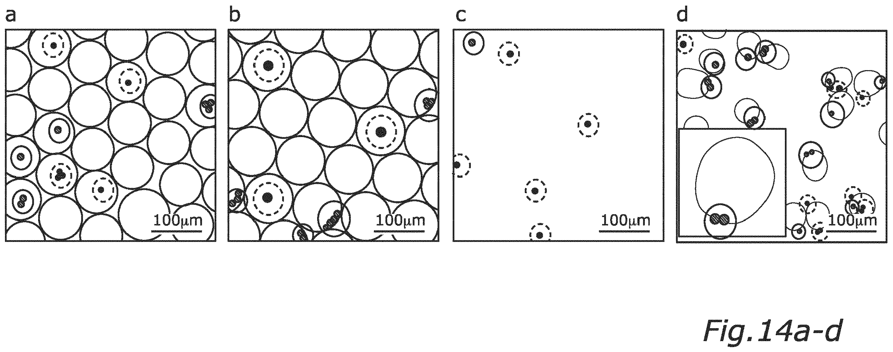

Fig. 14a-d: Microscope images demonstrating the fluorescence-based sorting process described in Example 8. A mixture of differently stained J774A.1 cells were sorted, thereby separating a plurality of said cells stained with Fluorescein (green, encircled with a white dashed line) from a plurality of said cells stained with ROX (yellow, encircled with a white solid line);

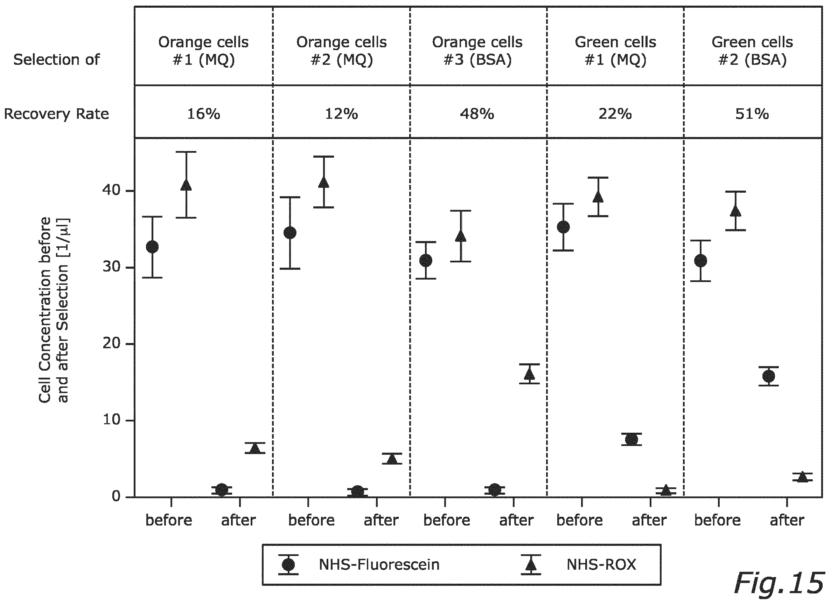

Fig. 15: Assessment of the recovery rate in the fluorescence-based sorting method presented in Example 8;

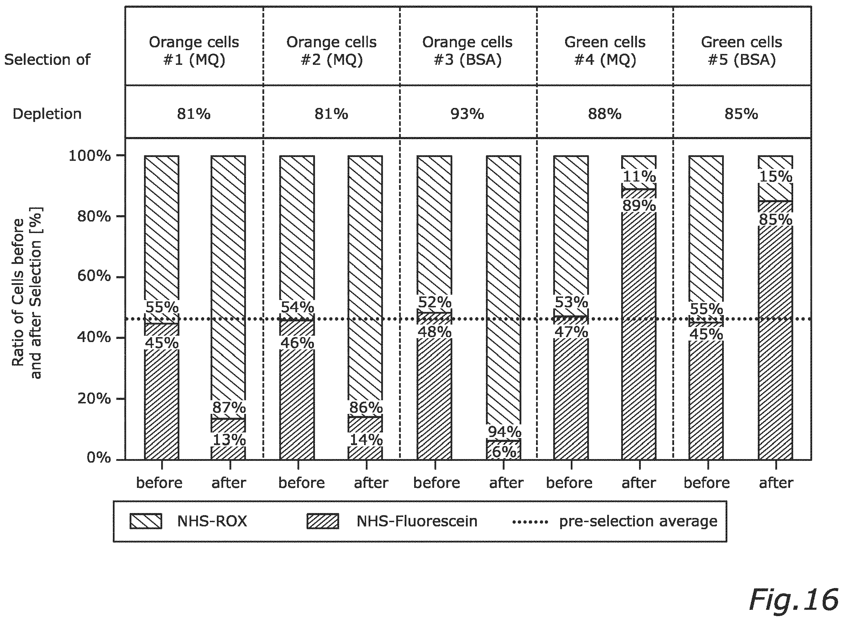

Fig. 16: Assessment of the depletion in the fluorescence-based sorting method presented in Example 8;

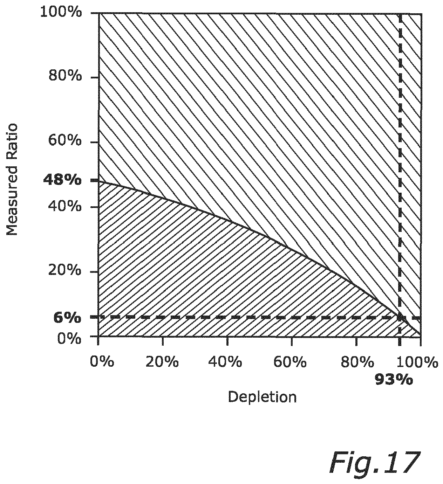

Fig. 17: Plot of the correlation of measured ratio to depletion in the fluorescence-based sorting method for the initial ratio of a sample presented in Example 8 (sorting run #3);

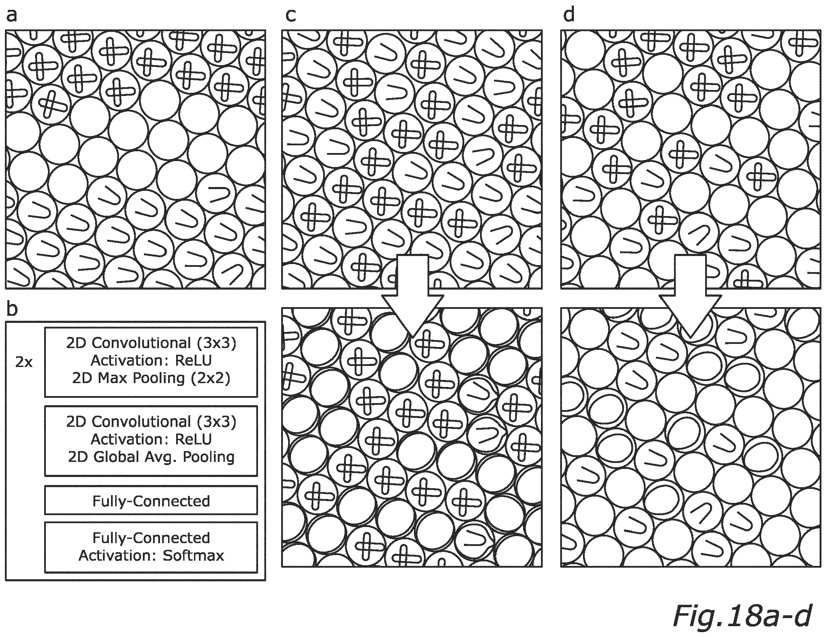

Fig. 18a-d: Demonstration of an image-based sorting method presented in Example 9;

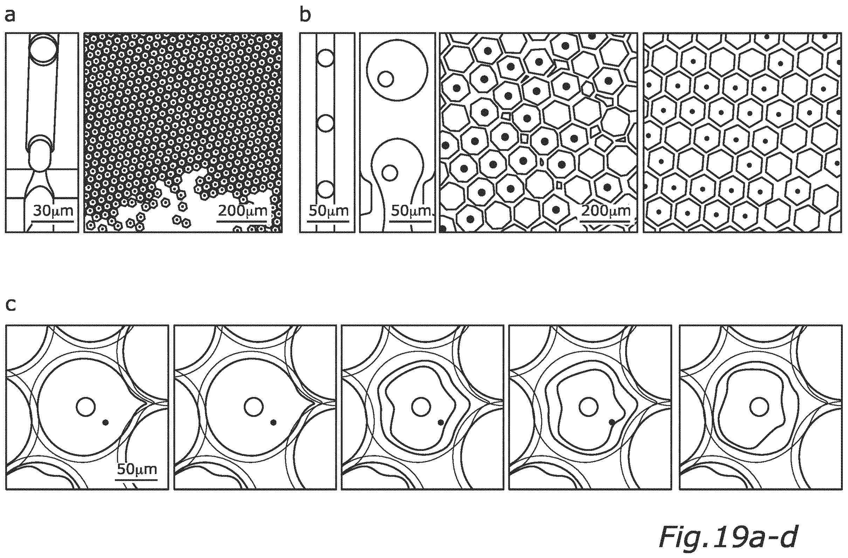

Fig. 19a-c: Production of double emulsion and printing of the oil droplets;

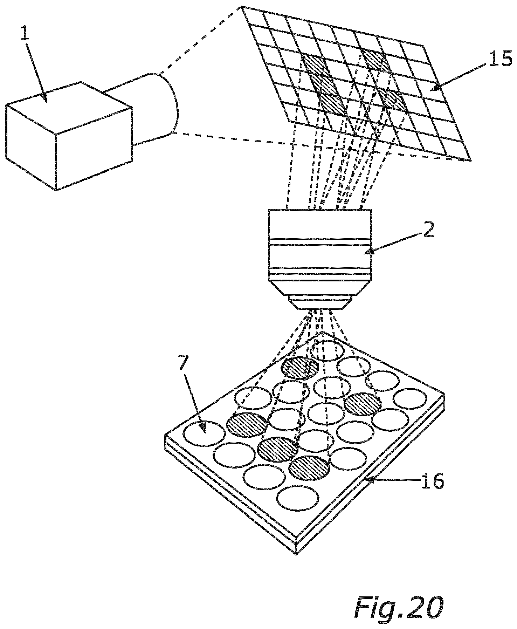

Fig. 20: Illustration of a method according to the present invention using a DMD; and

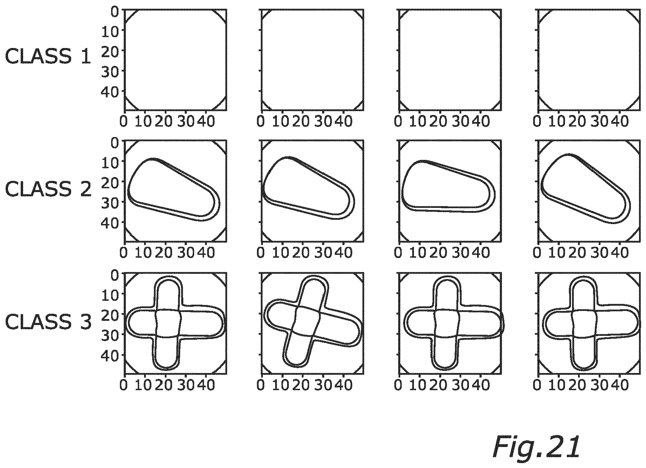

Fig. 21: Selected examples of CNN training data showing cropped images having 50x50 pixels which were fed into the network.

Fig. 1 shows an example setup for steps B) and C) of the method according to the present invention. The dispersion 6 comprises a plurality of components 4 which are aligned in a single layer in an aqueous phase inside a chamber, i.e., well. Herein the components are not encapsulated inside a compartment or a double emulsion. Therefore, the continuous phase of the dispersion (herein: aqueous phase) is polymerizable by comprising a photoresist (herein: water-soluble photoresist). It is also possible that the continuous phase of the dispersion is non-aqueous, e.g., oil-based, which would then require the use of a respectively soluble photoresist, e.g., oil-soluble photoresist. By polymerizing and thereby curing a part of the dispersion at least partially surrounding a compound of interest, at least one part of the resulting cured component may be separated from an at least one part of the components which are not cured. That way, a differentiation of the components inside the dispersion is possible which is required for the subsequent separation step. Within the plurality of components, at least one part is desired and a further at least one part is undesired. For example, the darker illustrated components are desired, whereas the brighter illustrated components are undesired. The invention is not limited thereto. It is also possible that the brighter illustrated components are desired, whereas the darker illustrated components are undesired.

In the embodiment as shown in Fig. 1 it suffices that at least one part of the photoresist surrounding the component is cured, e.g., a half of the component can be surrounded by a cured photoresist and another half of the component can be surrounded by a non-cured photoresist. In such a case, the photoresist surrounding the other components may be cured in this manner in order to obtain an approximately similar sedimentation rate of the components in the separation step. This can facilitate the separation of the plurality of components of interest from the rest. However, this is not limiting. A plurality of components with different curing extents of the surrounding photoresist may still be sufficiently separated since they may have an already different sedimentation rate than those components which are surrounded by completely non-cured photoresist.

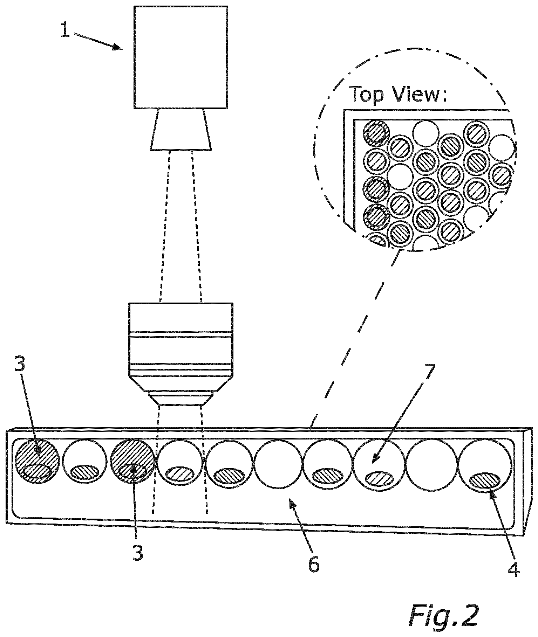

The general principle is that the components are analyzed, preferably autonomously by means of a microscope comprising an objective 2 and the polymerization of the photoresist is triggered by targeted illumination by means of a light source 1, preferably with light having a wavelength of from 365 nm to 405 nm, more preferably of 365 nm. More generally, the components of interest optionally encapsulated in a compartment are analyzed (step B)) in order to be separated from the remaining components (step D)) by means of a targeted illumination of the photoresist (step C)). This general principle described for Fig. 1 also underlies the example embodiment shown in Fig. 2, Fig. 3 and Fig. 4, which are described in the following.

Fig. 2 shows an alternative setup for implementing steps B) and C) of the method according to the present invention. In Fig. 2, each component is encapsulated in a water-in-oil droplet, wherein the droplets are embedded in a dispersion with the continuous phase thereof being an oil-phase, i.e., non-aqueous. The droplets further comprise a water-soluble photoresist. It is also possible that the continuous phase of the dispersion is aqueous which then would require oil-in-water droplets encapsulating oil-soluble components and oil-soluble photoresist. The use of droplets defines a specific local distance between one another which may facilitate and enable a reliable separation of one another by selective curing of the droplet content.

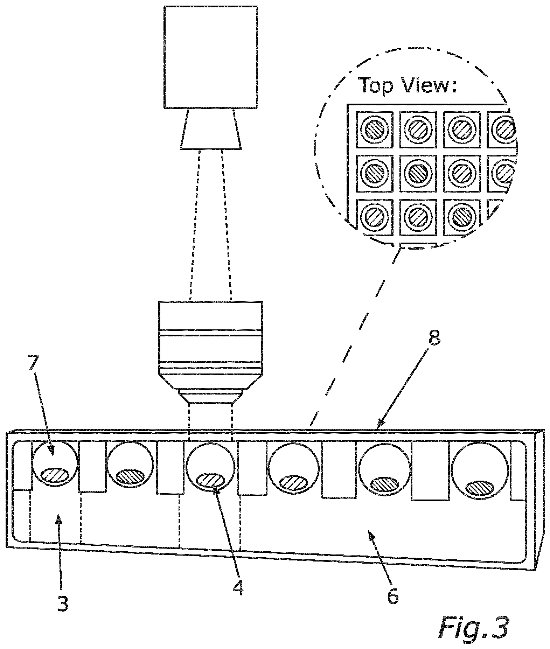

Fig. 3 shows an alternative setup for implementing steps B) and C) of the method according to the present invention. Fig. 3 is different from the embodiment shown in Fig. 2 in that the water-in-oil droplets are embedded inside a selection chamber 8. The selection chamber 8 may be configured to contain at least one droplet per well, preferably one droplet in a well as shown in Fig. 3. The application of a selection chamber 8, such as a 96-well, may further improve the separate localization of each droplet, which may facilitate and enable a reliable separation of one another by an even more selective curing of the droplet content. The selective curing mainly results from the chamber walls surrounding at least one droplet, thereby at least substantially immobilizing the droplet. Therefore, the light source can be more selectively punctuated towards the at least substantially fix location of the droplet.

Of course, depending on the size of the droplets, differently meshed chambers may be used. For smaller droplets, an appropriate immobilization thereof may be obtained by choosing a selection chamber having respectively small chambers, i.e., wherein the walls of one chamber may enclose at least one droplet (at least one droplet per chamber). For larger droplets, an appropriate immobilization thereof may be obtained by choosing a selection chamber having respectively large chambers, i.e., wherein the walls of each chamber may enclose at least one droplet (at least one droplet per chamber). The length of the chamber walls might also vary, i.e., it may be longer or shorter than shown in Fig. 3, which may depend on the kind of compartments and/or components used in the setup.

Of course, it is also possible that in the exemplary embodiment shown in Fig. 3 there are no droplets but components (i.e., components not encapsulated in droplets). The principle using a selection chamber as exemplarily described above for Fig. 3 may then apply mutatis mutandis for a dispersion comprising components not encapsulated in compartments.

Fig. 3 is further different from Fig. 2 in that the photoresist is comprised in the continuous phase (herein: oil phase), i.e., the photoresist is not inside the droplets as shown in Fig. 2. The application of a light for curing said photoresist may herein not lead to a polymerization inside the droplets (as shown in Fig. 2) but to a polymerization of the droplet surrounding environment, i.e., a part of the oil-phase embedding the droplets. The oil phase herein may therefore also be referred to as a "polymerizable oil phase" as it comprises the photoresist. This strategy might facilitate the preparation of the droplets since they would not need to comprise the photoresist. This strategy might also be beneficial when using a component which may interact, chemically react and/or decompose in the presence of a photoresist. Of course, it is possible to combine the strategies shown in Fig. 2 and Fig. 3, if appropriate, i.e., wherein the photoresist is comprised in the droplet and in the continuous phase (not shown). Of course, it is also possible to implement a setup as shown in Fig. 3 with the difference that the continuous phase is an aqueous phase. The latter may require that the compartments encapsulating the components are filled with a non-aqueous phase.

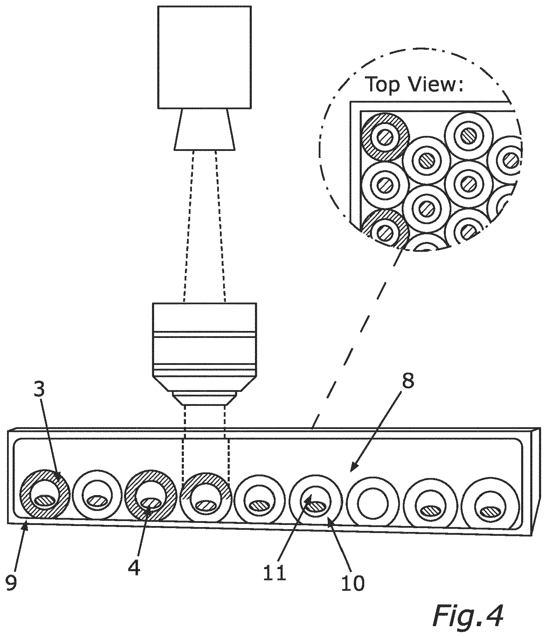

Fig. 4 shows an alternative setup for implementing steps B) and C) of the method according to the present invention. The dispersion comprises an aqueous phase as continuous phase and double emulsions, i.e., W/O/W droplets, encapsulating the components. The outer shell 10 (herein: outer oil shell) of the double emulsion comprises the photoresist. After insertion into a chamber the double emulsions sink to the bottom of the chamber and arrange on a grid, as shown in Fig. 4. This strategy is beneficial in order to avoid a contact of the components with the photoresist. That way, the setup shown in Fig. 4 may be used for when a component is sensitive towards the photoresist, i.e., a component which may interact, chemically react and/or decompose in the presence of a photoresist.

The setup shown in Fig. 4 might alternatively be implemented by providing a dispersion comprising double emulsions, wherein the double emulsion is an O/W/O droplet (oil-in-water-in-oil droplet). In other words, the outer shell of the double emulsion is based on an aqueous phase, the inside of the double emulsion is based on a non-aqueous phase (e.g., oil), and the continuous phase of the dispersion is also a non-aqueous phase (e.g.., oil).

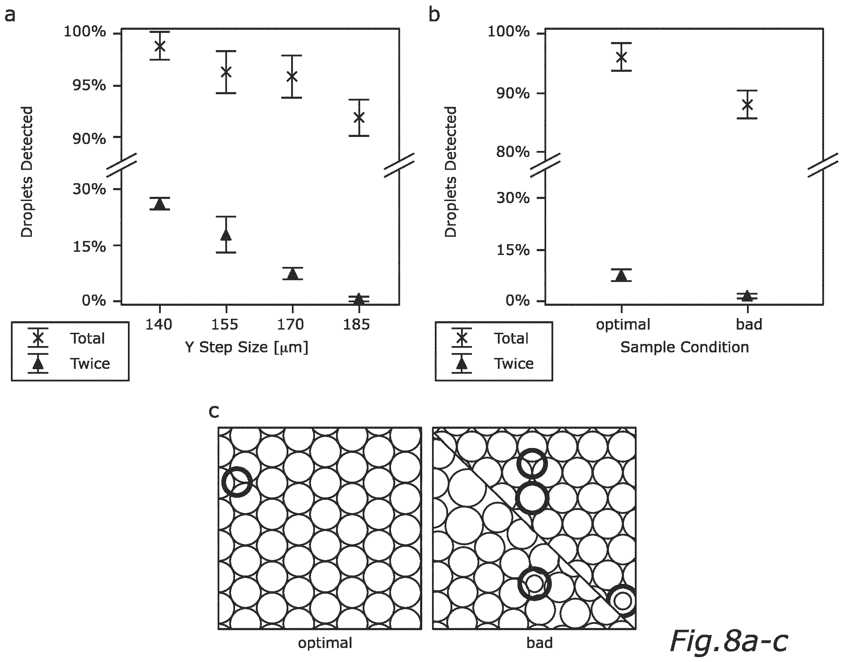

Fig. 8 shows images taken for demonstrating the accurate circle detection performed for the droplets used in the method according to the present invention. In case a microscope is used for analyzing the components optionally encapsulated in compartments, the threshold parameters for circle detection need to be fine-tuned to the specific set-up of the microscope. Unfocused droplets, e.g., due to moving of the droplets, greatly decrease the detection quality. In order to avoid the uncontrolled mobility of the droplets during the scanning process, the chamber slide comprising the droplets (and/or the components) must be well secured. Nonetheless, the surface (i.e., plane) of the droplet layer may be re-determined at certain intervals during the scanning process in order to maintain the accuracy of circle detection. Furthermore, a more uniform sample allows an improved circle detection. The provision of mono-disperse droplets may for example be achieved by producing the droplets using the microfluidic chap technique. Also, to combat the failed detection of droplets that are partially out of scanning frame, the target area can be divided into overlapping instead of adjacent tiles. This is especially significant for imaging at high magnification with a 40x objective, when two rows and three columns of droplets barely fit in the small field of view.

Fig. 8a shows the fraction of droplets detected as a function of the step size in y-direction of successively acquired tiles. The step size in y- direction for two frames (i.e., tiles) can be defined as the physical distance in y-direction of two points on the sample that are imaged to the same pixel of the two frames, respectively. The y-direction is chosen to be parallel to the top margin of the frame. The overlap of imaged tiles is decreased by increasing the step size in y-direction. Increasing the step size reduces the amount of successfully detected droplets (at a step size of 170 $\mu$m there is no overlap). The increased detection accuracy for small step sizes comes at the cost of droplets getting detected twice increasing the overall sorting time.

Fig. 8b shows the fraction of droplets detected under two different conditions. The detection accuracy suffers from a high amount of poly-disperse or stacked droplets obscuring droplets beneath or above them. Representative images for the conditions are shown in Fig. 8c.

Fig. 12 shows a software architecture designed for use with the Zeiss microscope having a software extension allowing scripting in the language of Iron Python 2.7 which was used in Example 6. Alternatively, another microscope, such as by Nikon, and/or another language, such as a C-like programming language may be used. The scripts written in Iron Python in the macro environment issue commands to the microscope and communicate with a local HTTP server, that allows more sophisticated data analysis. The macro sends out an HTTP request with the necessary information attached to one of the routes (in curly brackets), the data is evaluated by the server and the results sent back to the macro. For example, for the detection of droplets a request is sent to /detectdroplets with the file-path of the image attached to the body, and the response will contain the coordinates and diameters of the detected droplets.

Fig. 13 visualizes an example flow chart of a script executed in the selection according to step C) used in Example 6. After putting a slide containing the droplets on the stage of a microscope as shown in step 5 of Fig. 11b, the user has to select the target area in which droplets will be scanned and potentially polymerized by illumination, by setting the positions of its corner points. Afterwards, the plane in which all droplets are in focus has to be determined. Having all the droplets focused precisely at their equatorial plane is important for the circle detection. If the focus is off by merely 5 $\mu$m, the droplets appear blurred and the accuracy of the circle detection, which is optimized on in-focus droplets, decreases significantly. Using the autofocus for each scan would take a significant amount of time and the ZEN-integrated autofocus does not work reliably with droplets. Therefore, the surface of the droplet plane must be manually determined beforehand. The collected focus positions are sent to the server via the /setfocus route which determines an interpolator function using the LinearNDInterpolator from the scipy package based on Qhull [2]. The interpolator is saved and can subsequently be accessed from the macro by sending the current coordinates of the stage to the server through the /getfocus method, which will apply the interpolator and return the appropriate focus position. The macro prepares the microscope by removing the condenser, which improves circle detection and sets the analog gain to its maximum setting to minimize the required transmission light intensity to limit the overall exposure of the photoresist to light. Then, it divides the target area into adjacent tiles, such that every part of the area will be imaged, and moves the stage to view the top-left tile to start the selection process. For each tile, the microscope repeats the following steps: It adjusts the focus for the current tile by requesting it from /getfocus. It then acquires an image and sends the file-path to /detectdroplets on the server, which loads the image and executes a script regarding detection of droplets of interest. Here, the center coordinates and radius of all visible droplets are detected using Hough Transform provided by the OpenCV package [3]. The results are returned to the macro, which moves the stage to center each of the visible droplets sequentially for analysis. During the analysis, the stage height is adjusted to focus on the lower third of the droplets, because the cells sink to the bottom. Each droplet is imaged under fluorescence or bright-field illumination and then sent to the server via /scancircle or /shapedetection, which refers to a script regarding scanning circles and/or shape detection. For fluorescence detection within a circle, the image from the respective fluorescent channel is masked with a circle of the provided droplet radius and the number of pixels within that circle with an intensity above a certain threshold are counted. For shape detection, the image is cropped to a small rectangle at its center and the pattern is predicted by feeding the cropped image into a pretrained convolutional neural network. The macro then collects the results of the droplet evaluation for the current tile and selects the DAPI reflector, if undesired targets have been identified. This allows the microscope to polymerize unwanted droplets, by sequentially centering them in the field of view and briefly (500 ms) turning on the 365 nm LED at 23 %. This intensity was chosen to enable fast printing of the targeted droplet without polymerizing the neighboring droplets. Afterwards, the stage moves to the next tile and, if necessary, re-selects the reflector required to detect droplets containing the unwanted fluorescence signal. The sample is then scanned line by line, reversing direction after each line to minimize unnecessary stage movement. This way droplets in the entire chamber are selected.

Fig. 20 shows the illumination by the epifluorescence light source being processed by the digital-micromirror-device (DMD) and the desired pattern projected onto the sample through the objective lens. As depicted in Fig. 20, this may be used to target and polymerize individual droplets simultaneously from an array of droplets, which may significantly speed up sorting. About 5000 100 $\mu$m and 50 000 30 $\mu$m droplets can be imaged at once using a 1$\times$ objective lens, which would allocate 11 $\times$ 11 pixel and 4 $\times$ 4 pixel per droplet on a standard DMD with a 1140 $\times$ 910 pixel resolution. Assuming every droplet contains a cell and each tile needs around 10 s for detection and polymerization as is currently the case at 40$\times$, this would imply sorting rates of 500 cells/s and 5000 cells/s.

The following further exemplifies the present invention and provides experimental details. It is to be understood that the present invention is not limited to these details below, even though they may be regarded as preferred embodiments, either taken alone or in combination with each other.

## Reagents and Materials

In the present invention, a water-soluble photoresist was used for the production of w/o droplets, and an oil-soluble photoresist was used for the production of the double emulsion.

The water-soluble photoresist consisted of the monomer being polyethylene glycol) diacrylate (PEGDA, Mw $\approx$ 575), and the photoinitiator being lithium phenyl-2,4,6-trimethylbenzoylphosphinate (LAP), both of which were purchased from Sigma-Aldrich.

The oil-soluble photoresist consisted of the monomer being 1H,1H,2H,2H-tridecafluoro-n-octyl acrylate (stabilized with HQ + MEHQ), the cross-linker being 1,6-bis(acryloyloxy) -2,2,3,3,4,4,5,5-octafluorohexane (stabilized with MEHQ) and the photoinitiator being 2,2-dimethoxy-2-phenylacetophenone, all of which were purchased from TCI Chemicals. For the oil-phase, the fluorinated oil Novec 7500 (3M) was used.

## Cell culture preparation and staining

After fixation in ice-cold ethanol under vortexing, J774A.1 mouse macrophages were resuspended in Dulbecco's phosphate-buffered saline (Gibco DPBS, ThermoFisher) with 2 wt% bovine serum albumin (BSA, Albumin fraction V, Carl Roth) for at least 12 h. The cells were washed twice in DPBS and stained with ROX NHS-Ester (Rhodamin-X, Lumiprobe) or NHS-Fluorescein (5/6-carboxyfluorescein succinimidyl ester, Thermo Fisher Scientific). After 60 min, the cells were washed with DPBS five (5) times to remove the remaining dye. The cells were then resuspended in a PEGDA dilution containing PEGDA (Mw $\approx$ 575, Sigma-Aldrich) diluted in Milli-Q to 50 vol% with 20 mg/mL LAP, thereby providing an aqueous phase comprising J774A.1 cells.

## Production of Microfluidic Chip using Soft Lithography

Two-part SYLGARD® 184 silicone elastomer base and curing agent (DOW Europe) were mixed in a 9:1 weight ratio, poured onto a master mold and cured in an oven at 70 °C for 3 h. The resulting cured PDMS (polydimethylsiloxane) was removed from the master mold and inlets were created using a 0.5 mm biopsy puncher (Harris-Unicore). The PDMS blocks were sonicated in ethanol for 5 min. Then, 24 $\times$ 50 mm$^2$ high precision microscope cover glasses (Carl Roth GmbH + Co. KG.) were sonicated in 200 mL Extran® MA 02 (EMD Millipore Corporation) and 400 mL demineralized water for 15 min, and afterwards sonicated in pure demineralized water for 15 min. Both were thoroughly dried with nitrogen and placed in a plasma cleaner and exposed to oxygen plasma at 0.07 mbar and 200 mW for 30 s. The PDMS was bonded to the cover glass immediately after and baked for 1 h, thereby providing a PDMS-based microfluidic chip (also referred to as "microfluidic chip" or "PDMS chip").

## Production of Photopolymerizable W/O (water-in-oil) Droplets

The microfluidic chip was coated with Ombrello (Toulifly), which was inserted using a 1 mL Injekt-F Luer Solo syringe (B. Braun) and PTFE tubing (BOLA). Then, 1.43 wt% of 008-FluoroSurfactant (RAN Biotechnologies) was added to Novec 7500 (3M) for the oil phase. The aqueous phase comprising J774A.1 cells was obtained as described above under "Cell Culture Preparation".

Both phases (oil phase and aqueous phase) were inserted into the PDMS chip using 1 mL Luer syringes (BD Plastipak) and PTFE tubing. W/O droplets were then collected in an Eppendorf tube (Eppendorf).

## Production of Photopolymerizable Double Emulsion: W/O/W (water-in-oil-in-water) Droplets

The production of a double emulsion was achieved by a method using a single microfluidic chip (Method A) or a microfluidic chip assembly of at least two parts of a microfluidic chip (Method B). For both methods, the channels of the microfluidic chip(s) were coated in order to ensure the wetting of the outermost phase at the respective droplet fabrication step. For the hydrophilic coating in Method A or B, the assembled microfluidic chips were exposed to oxygen plasma for 1 min at 0.07 mbar and 100 mW, and subsequently flushed with an aqueous solution of 5 wt% poly(vinyl alcohol) (PVA, Mw 13 000-23 000, Sigma-Aldrich). After 10 min, the channels were flushed with air and the microfluidic chip was baked at 105 °C for 15 min.

Milli-Q with 0.075 wt% methylene blue (Sigma-Aldrich) was used for the inner phase, while the middle phase consisted of 50% Novec 7500 with 1.43% 008-Fluorosurfactant and 50 % of the oil-soluble photoresist listed above, using a monomer to cross-linker ratio of 5:1, and 16 mg/mL photoinitiator, whereas the outer phase Milli-Q contained 0.075 wt% methylene blue and 5 wt% PVA.

## Method A: Single Microfluidic Chip

The production of a double emulsion performed in a single microfluidic chip with two sequential junctions is well known to the skilled person, for example from Ji-Won Kim, Sang Hoon Han, Ye Hun Choi, Wahyu Martumpal Hamonangan, Yoonjin Oh, and Shin-Hyun Kim. Recent advances in the microfluidic production of functional microcapsules by multiple-emulsion templating. Lab Chip, 22:2259-2291, 2022.

## Method B: Microfluidic Chip Assembly

First, W/O droplets of 30 $\mu$m diameter were produced at a cross-junction in a first microfluidic chip with a hydrophobic coating on the channel walls and a surfactant added to the oil-phase, as described above in "Production of Photopolymerizable w/o (water-in-oil) Droplets".

The droplets were then collected and sent through the main channel of a second chip with the same channel layout and hydrophilic coating, where the outer aqueous phase containing polyvinyl alcohol (PVA) was connected to the side channels. Here, stable W/O/W droplets with a thin oil shell were created, as depicted in Fig. 18a. The syringe controlling the W/O droplet phase in the main channel was set up vertically with the opening on top, causing the W/O droplets to float up the syringe and become tightly packed in the tube and channels. This method worked reliably and produced droplets with an oil shell of around 2 $\mu$m.

A thicker shell was produced by connecting the outlet of a microfluidic chip with 30 $\mu$m nozzle width and hydrophobic coating to a chip with a 80 $\mu$m nozzle and hydrophilic coating via tubing. In the first chip W/O droplets were produced, which were flowing straight into the main inlet of the second chip. Here, the outer aqueous phase was connected to the side channels and W/O/W droplets were produced at the junction. The spacing of the W/O droplets from the first chip could be controlled by changing the flow rates of the aqueous and oil phase in the first chip and thereby allowed to set the right flow ratio to ensure a single core per W/O/W droplet. The flow stabilized after about a minute, W/O droplets would arrive consistently and a double emulsion was produced.

## Release of cells from the droplets

### W/O Droplets

J774A.1 cells were released from the W/O droplets by demulsification of the droplets comprising the cells in an Eppendorf tube by using a Zerostatic 3 (Milty) anti-static gun, thereby placing two fingers behind the tube opposite of the tip of the gun and activating the gun repeatedly.

## Example 1 - Curing the Photoresist

As a proof-of-concept, the printing behavior, i.e., curing behavior, of the water-soluble photoresist described above (PEGDA and LAP) was investigated in W/O droplets and in a chamber, respectively. The photoresist was comprised in the W/O droplets in order to determine curing thereof inside the droplet. The chamber represented an alternative case for printing of a component not encapsulated in a compartment, i.e., the component is embedded in the continuous phase of the dispersion inside a chamber.

It should be noted, that in contrast to Example 1, the procedure described in Example 3 is directed to imprinting of single W/O droplets. The conditions described in Example 1 were rather roughly chosen (in contrast to Example 3). Said conditions offer imprinting of a series of droplets in an illuminated area, whereas the conditions developed in Example 3 are directed to a targeted imprinting of a single droplet.

### Procedure

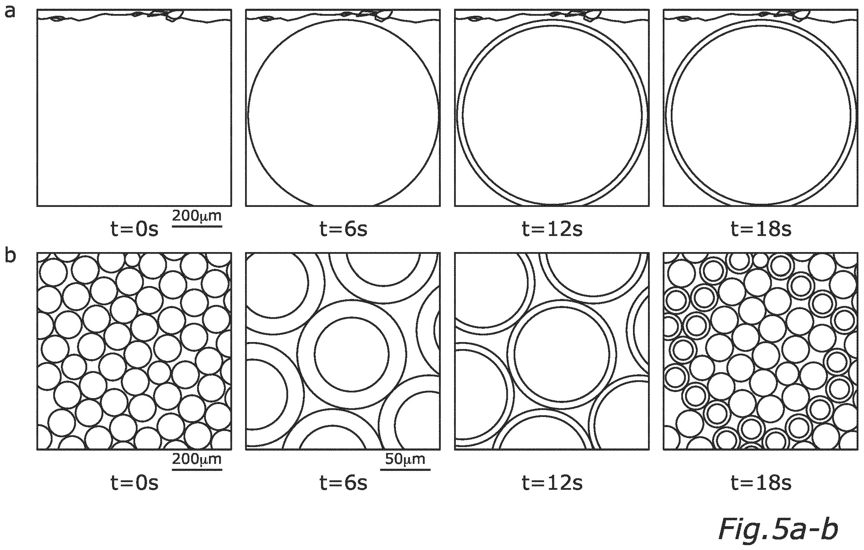

Curing, i.e., printing, the photoresist consisting of 50 vol% PEGDA dissolved in water with 20 mg/ml LAP was performed upon illumination with 365 nm light (2 % intensity) using the 40$\times$ objective and the field aperture in the fluorescence module fully opened. These conditions, in particular the aperture being fully opened at a 40x magnification, leads to the illumination of a series of droplets, see Fig. 5.

### Curing "outside" droplets

In case of curing "outside" droplets, i.e., curing of a photopolymerizable continuous phase of a dispersion comprising a component which is not encapsulated, or curing of a photopolymerizable solution comprising the photoresist, the photoresist in the respective illuminated area polymerized uniformly, see Fig. 5a.

Curing inside droplets

Polymerization within each droplet positioned in the illuminated area starts from the top of each droplet (t = 6s), expanding over time (t = 12s). The droplets at the center of the illuminated area eventually fully polymerize, while droplets in the periphery, where the intensity is lower, have only partly polymerized (t = 18s), see Fig. 5b.

**Example 2** - **Imprinting Droplets with Patterns**

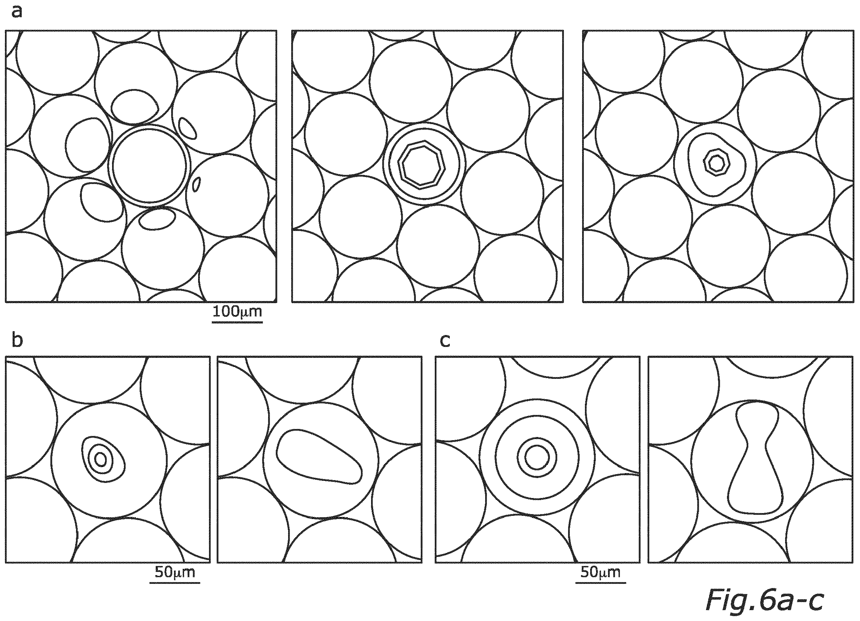

W/O droplets were imprinted as presented in Fig. 6a-c. That way, the determination of the precision of the Zeiss Imager M1 to print in W/O droplets was possible. The W/O droplets contained the water-soluble photoresist (50 vol% PEGDA dissolved in deionized water with 20 mg/ml LAP) and printing was initiated upon illumination with 365 nm light at high intensity (100%). The field aperture in the fluorescence module was closed as much as possible to minimize the illuminated area.

Fig. 6a shows an objective with higher magnification being able to polymerize smaller structures, as shown for different magnifications 10x (left), 20x (center) and 40x (right), respectively. The printed structure was an imprint of the fluorescence aperture, the illumination time was 500 ms. For 20x (center) and 40x (right) magnifications, polymerization was also visible around the imprint which could be avoided by reducing the intensity or illumination time. At 10x magnification (left), the imprint had a diameter of 150 $\mu$m, at 20x 60 $\mu$m and at 40x 30 $\mu$m.

The left-hand image of Fig. 6b shows focused on the equatorial plane of the droplet at $40\times$ magnification the center of a droplet being illuminated for 100 ms. The right-hand image of Fig. 6b shows the printed structure which sank to the bottom of the droplet and tipped over, revealing a comet-like shape of the printed column. The top of the structure (now at 4 o'clock) showed a distinct edge, the bottom got wider and fuzzier (now at 10 o'clock). A similar procedure was performed in order to obtain the W/O droplets imprinted with comet structures for their application in Example 9.

Fig. 6c shows illumination of the center of a droplet with a Zeiss LSM 900 confocal microscope (left) revealing an hour-glass-like shape of the printed structure (right).

**Example 3** - **Imprinting single droplets**

In order to deplete a sample of droplets optionally encapsulating undesired components, these droplets may be polymerized (i.e., cured, printed) according to the method according to the present invention. Therefore, precise targeting of individual droplets is beneficial. According to Fig. 6 this should be possible for droplets of 100 $\mu$m diameter, when using a $20\times$ or $40\times$ objective. However, when the field aperture in the fluorescence module is adjusted in an attempt to polymerize a single droplet, the photoresist in surrounding droplets starts polymerizing, even before the targeted droplet is fully polymerized. On the one hand, only by partially polymerizing the targeted droplet, the surrounding droplets can remain unaffected. On the other hand, partial polymerization may lead to microparticles in the droplet not getting successfully embedded in the hardened structure.

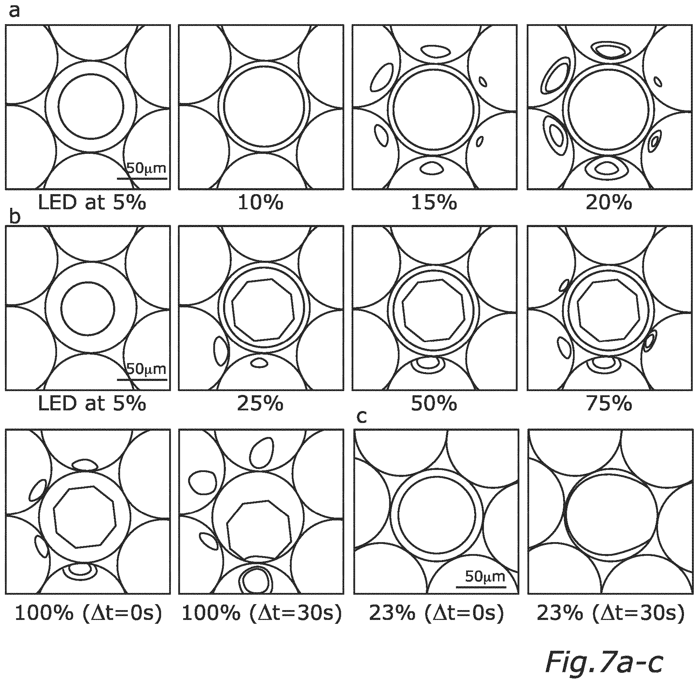

Herein, optimal conditions for precise single droplet imprinting was achieved by using 23 % intensity of the illumination light, 500 ms illumination duration, and the aperture being 1/8th opened, see Fig. 7c. The results obtained during the development of said optimal conditions are as follows.

Fig. 7 shows microscope images in the development of optimal conditions for imprinting single W/O droplets.

Fig. 7a shows the intensity of the illuminating light being gradually increased to the right, while the fluorescence field aperture and illumination time were fixed. For the targeted droplet in the center, the portion that was polymerized increased with higher intensity, but before the entire droplet diameter was reached, the neighboring droplets began to show polymerization (15 %). This was also the case when increasing the illumination time or fluorescence field aperture instead.

Fig. 7b shows a droplet printing when using small fluorescence field aperture and high illumination intensity which resulted in large portions of the targeted droplet being polymerized, while minimizing polymerization of photoresist in surrounding droplets. The fluorescence field aperture and illumination time were fixed and the intensity was increased to the right. With the field aperture only slightly being opened, the shape of the aperture was imprinted into the droplet, leading to a non-uniform polymerization. The non-uniform polymerization caused the structure to tip over, as depicted for 100 % illumination. $\Delta$t: time after polymerization.

Fig. 7c shows a small fluorescence field aperture which decreased the signal of fluorescent objects in the droplet. As the field aperture was not motorized, a compromise for the optimal conditions was used for the selection experiments (23 % intensity, 500 ms, aperture 1/8th opened), where neighboring droplets did not polymerize and fluorescent cells were visible. The latter is beneficial for the sorting process in order to screen components, such as cells, in order to identify and separate them.

**Example 4** - **Circle Detection**

In order to accurately determine the shape of the analyzed sample, such as components optionally encapsulated in compartments, the shape detection needs to be improved. In particular, using image-based sorting methods which is an embodiment of the herein described invention requires an accurate circle detection, e.g., of the analyzed cells and/or droplets. Fig. 8a exemplarily illustrates how an accurate circle detection of W/O droplets was achieved for the method according to the present invention.

As shown in Fig. 8a, for a higher overlap of tiles the detection accuracy increased. However, a high overlap also raised the number of droplets that got detected twice, and therefore increased the duration of the whole selection process. When the scan pattern consisted of adjacent tiles, $(96 \pm 2)$ % of droplets were detected under optimal conditions, that is, mono-disperse droplets with only single stacked droplets, and $(7 \pm 1)$ % of droplets were detected twice. Under bad conditions, such as when there are different droplet sizes and multiple, adjacent stacked droplets, the accuracy decreased to $(88 \pm 2)$ % with $(2 \pm 1)$ % of droplets getting detected twice (compare Fig. 8b and Fig. 8c).

The detection accuracy e is defined as the fraction of compartments and/or components that is correctly identified by the mechanism compared to the number of compartments and/or components imaged:

$$e = \frac{n_{identified}}{n_{imaged}}$$

Effect of the condenser illumination

It was further found that the circle detection may be improved by excluding the condenser lens from the transmission light path. In the absence of a condenser lens, the droplets were evenly illuminated and had a uniform appearance in the entire field of view. Sorting method using a microscope as described in the following Examples was therefore performed without using a condenser lens.

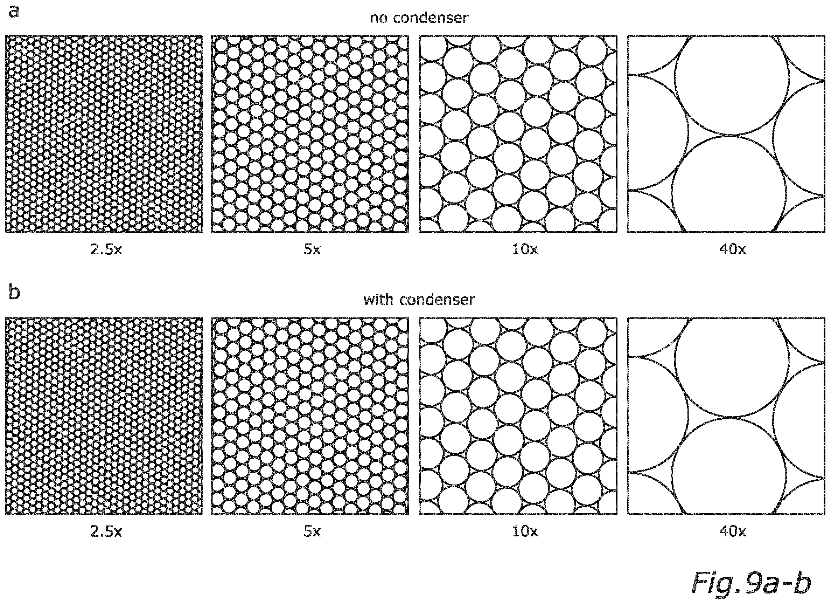

Fig. 9a shows that in the absence of a condenser lens all droplets were evenly illuminated and showed a similar appearance.

Fig. 9b shows that if a condenser lens was used droplets further off center of the field of view were unevenly illuminated and lost contrast, therefore the droplets appeared less uniform. The effect was stronger with decreasing magnification.

**Example 5** - **Chamber Prototypes**

In the interest of developing a cheap, self-made chamber to hold the W/O droplets, different chamber prototypes were tested. In any chamber where either the W/O droplets or oil (i.e., continuous phase of the dispersion being oil) were exposed to air, the fluid formed a meniscus and the W/O droplets did not align in a single plane which may be beneficial e.g. for sufficient circle detection. Rather, the droplets got stuck on the chamber walls. Therefore, an enclosed, sealed casing was developed in order to get the droplets to arrange in a single plane. The development of different chamber prototypes was as follows.

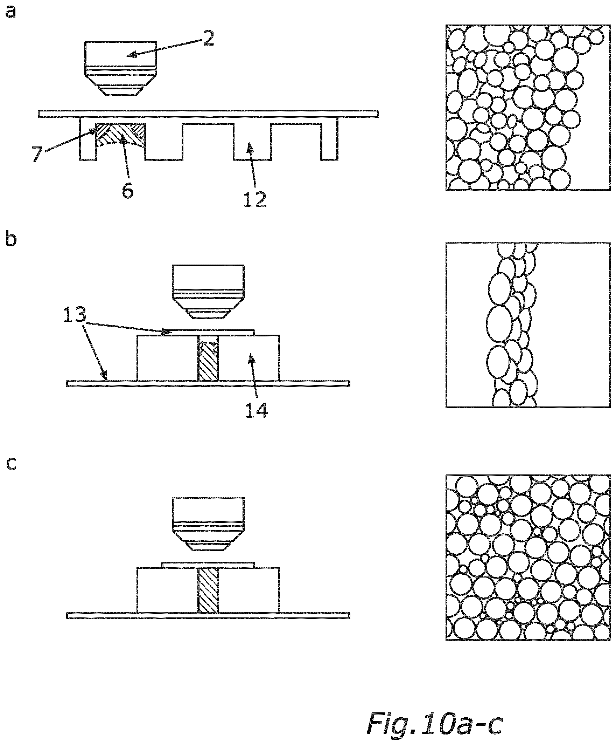

Fig. 10a shows that in an inverted standard 96-well plate or an Ibidi angiogenesis $\mu$-slide (cat.no. 81501) the droplets did not arrange in a single-layer plane at the top, but gathered and got stuck mainly on the side of the wells.

Fig. 10b shows that cutting a hole in a PDMS chip and filling it with oil (when W/O droplets are used), the droplets got stuck on the sides and did not arrange at the surface.

Fig. 10c shows an optimal layering of the droplets which is as described for Fig. 10b, wherein said hole was completely filled to the brink and topped off with a glass slide. As a results, air was avoided as much as possible inside the chamber in order to arrange the droplets in a single layer. In Example 6, a chamber model ($\mu$-Slide Luer from Ibidi) based on this concept was used, wherein the chamber was also filled to the brink with the continuous phase of the dispersion, thereby excluding air inside the chamber. The basins of the chamber used in Example 6 were then sealed.

**Example 6** - **Workflow of a Method according to the present invention**

An example workflow of the method according to the present invention was implemented. The single steps thereof are presented in Fig. 11a to Fig. 11d. The method was performed using the oil phase described above as continuous phase of the dispersion (fluorinated oil: Novec 7500 (3M)). In said dispersion, W/O droplets comprising J774A.1 cells and the water-soluble photoresist described above (PEGDA and LAP) have been resuspended. This setup is based on the one shown in Fig. 2.

Provision of a dispersion (Step A); Fig. 11a)

The single steps (steps 1 to 4) for the provision of said dispersion is shown in Fig. 11a. Fig. 11a is an embodiment of step A) of the method according to the present invention.

In step 1, a sterile chamber (herein: Ibidi Luer slide) was put on a flat surface and 30 $\mu$L of fluorinated oil Novec 7500 (3M)) was added in one basin, the oil having the same concentration of surfactant (008-FluoroSurfactant (RAN Biotechnologies)) as used for the oil used for W/O droplet production. In step 2, the slide was propped up to a 30 degrees angle and 10 $\mu$L of W/O droplets were added to the same basin. After two (2) minutes, all droplets were floating to the top of the oil away from the basin. In step 3, the slide was put back on a flat surface and the chamber was filled up with the fluorinated oil. No air was remaining in the chamber, and no oil was collected in the basins. In step 4, the two-component adhesive twinsil (picodent) was mixed in a separate container in order to seal both basins of the chamber by dripping it into them using a pipette tip. The resulting adhesive plug had a thickness of about 3 mm.

Scanning and Illumination of the Dispersion (Steps B) and C); Fig. 11b)

The single steps (steps 5 to 7) for the scanning and illumination of the dispersion is shown in Fig. 11b. Fig. 11b is an embodiment of steps B) and C) of the method according to the present invention.

In step 5, the inverted slide obtained in step 4 was put on a stage of a microscope equipped with a light source of 365 nm (Zeiss Axio Imager M1 having an IronPython 2.7 scripting software extension), such that one side was slightly elevated causing the droplets to slowly flow towards one basin and arrange in a plane without stacking. The slide was fixed in place in order to avoid shifting during the movement of the stage. In step 6, the coordinates were set of the corner points of the area in which droplets should be scanned. Focal positions for different positions were set and saved within an area from which a precise focal plane was interpolated by the script to keep the droplets in focus at all times of scanning time. In step 7, properties of the cells encapsulated in droplets were chosen and a respective script was run. Fig. 12 shows the software architecture used for the selection step C), and Fig. 13 shows the flow chart of the macro code used therein. According to the specific script, those components encapsulated in droplets fulfilling the properties chosen beforehand were selected for being illuminated in order to cure the photoresist inside said droplets encapsulating the undesired components of interest.

Separation (Step D); Fig. 11c and Fig. 11d)

The single steps (steps 8 to 15) for the separation of the cells of interest from the cells not of interest is shown in Fig. 11c and Fig. 11d. Fig. 11c and Fig. 11d are an embodiment of step D) of the method according to the present invention.

In step 8 of Fig. 11c, the partially illuminated slide obtained in step 7 was removed from the stage and put on a flat surface. The adhesive plug was removed from the basin opposite to where the droplets gathered in order to reduce the risk of losing the droplets. In step 9, 40 μL of fluorinated oil was added together with the surfactant (008-FluoroSurfactant (RAN Biotechnologies)) to the open basin. In step 10, the slide was propped up to a 30 degree angle with the open basin on the elevated side. One of the provided caps (included with Ibidi Luer slide) was added to the open basin in order to avoid evaporation of the oil and two (2) minutes were waited until the droplets left the chamber and formed a thin layer on top of the oil. In step 11, the droplets were collected using a 20 μL pipette. In step 12, the droplets were slowly pipetted into a 0.2 mL Eppendorf tube. Steps 9 to 12 were repeated at least once.

In step 13 of Fig. 11d, 42 μL of buffer was added to the tube with the opening of the pipette tip positioned in the oil, just beneath the droplets. That way, the droplets separated and the cells were held away from the water-oil-interface. In step 14, the cells were released by destroying the droplets using an anti-static gun. Two fingers were held right behind the tube, opposite to the antistatic gun in order to increase the breaking of the droplets. About 100 clicks were necessary to break the droplets. In step 15, the cured photoresist comprising mostly the cells not of interest would sink to the water-oil-interface. The aqueous phase comprising mostly cells of interest was mixed using a 20 μL pipette before collecting it. The opening of the pipette was close to the polymerized photoresist. In case an emulsion was formed during mixing, step 14 was repeated.

**Example 7** - **Fluorescence-based identification and separation of J774A.1 macrophage**

The method according to the present invention was not only successfully implemented but it was analyzed for recovery rate, depletion, and purity, which are described further below.

Experimental setup

A setup based on the one shown in Fig. 2 was used for the scanning and illumination steps B) and C) of the herein described method. As components, natural cells J774A.1 mouse macrophage encapsulated in photopolymerizable W/O droplets were used, i.e., droplets further comprising the photoresist. The encapsulation was performed by means of the microfluidic chip pathway as described above. For the continuous phase of the dispersion fluorinated oil Novec 7500 (3M) was used, as described above. The workflow described in Example 6 was used, wherein the cell concentration in a sample was chosen to be about 3000 cells per 10 μL of droplets (about 24% of droplets containing at least one cell). A sample containing a 1:1 ratio of droplets comprising NHS-ROX-stained cells to droplets comprising NHS-Fluorescein-stained cells was chosen. The samples were imaged using an 40x objective (Zeiss LD Achroplan 40x/0.6) and a macro scripted in Iron Python 2.7 connected with separate image analysis scripts in CPython 3 environment executed sorting out NHS-Fluorescein and NHS-ROX-labeled cells, respectively.

Sorting concept

Those droplets encapsulating Fluorescein-stained cells were chosen to be the components encapsulating components of interest, while the droplets encapsulating ROX-stained cells were chosen to be the components encapsulating components of no interest. During the scanning step, the sample comprising both species was analyzed for the droplets containing the undesired cells in order to illuminate these and hence cure the photoresist inside these droplets. In contrast thereto, the droplets comprising the desired Fluorescein-stained cells were not polymerized, i.e., printed or cured.

Accordingly, a separation of the differently stained cells was possible.

Sorting Procedure

J774A.1 mouse macrophages were fixed and stained with one of two different fluorescent dyes (as described above under "Cell Culture Preparation"), thereby obtaining cells divided into two populations: one group stained with NHS-Fluorescein (green, encircled with a white dashed line in Fig. 14 a to d) and one group stained with NHS-ROX (yellow, encircled with a white solid line in Fig. 14 a to d).

Before each sorting run, the ratio of encapsulated cells in the chamber was determined by imaging droplets in the bright-field and fluorescent channels (Zeiss Colibri, GFP: Zeiss Filter Set 38 HE, Cy3: Zeiss Filter Set 43 HE) at random positions within the chamber with the 10x objective (Zeiss Plan-Neofluar 10x/0.3); see Fig. 14a. The cells were counted by thresholding the images of the fluorescent channels using a custom FIJI [4] macro and the Analyze Particles plugin.

Using the Zeiss:IronPython2.7 (Axio Imager M1 and connected CPython3 Image analysis scripts) microscope system, droplets encapsulating the undesired cells were automatically identified and polymerized upon illumination with light of a wavelength of 365 nm (see Fig. 14b). The droplets were then diluted with Milli-Q water. Alternatively, the droplets were diluted using DPBS with 2 wt% BSA which is further described below under "recovery rate". As described in Example 6, the droplets were released, the undesired cells in cured photoresist quickly sank to the water-oil-interface and 26 µL of the diluted aqueous phase was transferred into an uncoated Ibidi µ-Slide VI chamber. The released desired cells are shown in Fig. 14c.

The entire chamber was imaged in tiles and the cells were counted with a custom FIJI macro and analyse particles' plug in. For each tile the respective concentrations were calculated considering the number of cells and true volume of the aqueous solution in the frame, that is number of droplets times average droplet volume for the images taken of droplets and volume of the chamber visible in the frame for the release. The cured photoresist spheres encapsulating the undesired ROX-stained cells are shown in Fig. 14d, wherein said cured photoresist spheres were imaged to review the rate at which cells were successfully embedded in a polymerized droplet.

Fig. 14a shows a plurality of W/O droplets encapsulating NHS-ROX (yellow, encircled with a white solid line) or NHS-Fluorescein-stained (green, encircled with a white dashed line) J744A.1 cells, and a further plurality of W/O droplets not encapsulating said cells. This image was taken before a scanning run of the sorting process in order to determine the ratio of encapsulated cells in the chamber.

Fig. 14b shows W/O droplets after the selection of Fluorescein-stained cells. The photoresist in droplets containing ROX-stained cells were polymerized. Right after printing, the polymerized structure were slightly thinner at the bottom of the droplet, where the cell was embedded. Over time, the presumably densest part of the structure sank to the bottom of the droplet: The structure reoriented itself and thereby lifted up the cell (positioned in the bottom right quarter of most polymerized droplets in the micrograph).

Fig. 14c shows an enriched dilution of Fluorescein-stained J744A.1 cells after ROX-stained J744A.1 cells have been sorted out.

Fig. 14d shows the droplets comprising undesired J744A.1 cells (ROX-stained, encircled with a white solid line) which were cured in order to separate these droplets from those comprising the desired J744A.1 cells (Fluorescein-stained, encircled with a white dashed line). Some Fluorescein-stained cells inadvertently stuck to the cured droplets. The magnified image at the left bottom of Fig. 14d shows ROX-stained cell embedded at the thinner side of the hardened droplet, scale bar 50µm.

**Example 8** - **Determination of sorting parameters**

Five sorting runs as described in Example 7 were performed for determining the sorting parameters of recovery rate, depletion, purity and throughput. The five different sorting runs were called 'Orange cells #1 (MQ)', 'Orange cells #2 (MQ)', 'Orange cells #3 (BSA)', 'Green cells #4 (MQ)' and 'Green cells #5 (BSA)', and are shown in Fig. 15 and Fig. 16.

a) Recovery rate

As used herein, the recovery rate r is a measure on how many desired components are lost during the entire sorting process and is defined as the percentage of components of interest that are recovered after the sorting run, compared to the number of target cells that were initially present in the sample:

$$r = \frac{n_{\text{ desired, isolated}}}{n_{\text{ desired, initial}}}.$$

The recovery rate was assessed by performing five sorting runs in order to separate the Fluorescein-stained cells from ROX-stained cells. In sorting runs #1 to #3 ROX-stained cells were desired and Fluorescein-stained cells were undesired. In sorting runs #4 and #5 Fluorescein-stained cells were desired and ROX-stained cells were undesired. The detailed procedure is described above.

After sorting, the desired cells were diluted as described above in Milli-Q solution or Dulbecco's phosphate-buffered saline (Gibco DPBS, ThermoFisher) with 2 wt% bovine serum albumin (Albumin fraction V, Carl Roth).

Fig. 15 shows that the recovery rate was increased when using the DPBS/3wt% BSA mixture for diluting the desired cells. Without being bound by theory, this may be explained in that the released cells attached to the cured droplets comprising undesired cells after release. This is also visible in Fig. 14d. As shown in Fig. 15, the two sorting runs where DPBS containing BSA is used for dilution the sample loss is reduced to around 50%.

The top row of Fig. 15 shows cells to be selected from mixture and medium used for dilution of the release in parentheses (MQ: Milli-Q water, BSA: DPBS with 2 wt% BSA). The middle row of Fig. 15 shows the recovery rate that is calculated from the amount of the wanted cell type that is present in the sample after selection as compared to before selection. The recovery rate is significantly increased if DPBS with BSA is used for dilution to keep free cells from sticking to the hardened droplets after release. The bottom row of Fig. 15 shows cell concentrations of both cells (Fluorescein-stained and ROX-stained, respectively) before and after selection, respectively. As the aqueous phase is diluted after release, the cell concentration before selection is shown as if it had been diluted in the same way to allow a clear assessment of the recovery rate.

b) Depletion

As used herein, the depletion d is defined as the fraction of undesired components that have been intentionally removed compared to the initial number of unwanted components:

$$d = \frac{n \text{ undesired, removed}}{n \text{ undesired, initial}}.$$

The depletion d was determined by comparing the ratio of the differently stained cells before and after selection by using the equation

$$d = 1 - \frac{f \cdot (1 - i)}{i \cdot (1 - f)}$$

where i and f are the fraction of the undesired cells before and after selection, respectively. The depletion is not calculated from the change in concentration as this would also include the general sample loss during the entire process, which also affects the desired cell population.

The top row of Fig. 16 shows cells to be selected from the mixture, with the medium used for dilution of the release in parentheses (MQ: Milli-Q water, BSA: DPBS with 2 wt% BSA). The middle row of Fig. 16 shows the depletion calculated from the measured change in ratio.

The bottom row of Fig. 16 shows the ratio of cells before and after selection, respectively. As deducible in Fig. 16, it was found that after each sorting run the ratio of cells had significantly shifted in favour of the desired group. Overall, undesired cells were successfully removed with an average depletion rate of $(85 \pm 5)$ % resulting in samples of $(88 \pm 3)$ % purity.

Fig. 17 shows the correlation of the final measured ratio (on the y-axis) to a hypothetical depletion rate (on the x-axis) for a sample initially containing 48 % undesired Fluorescein-stained cells (selection run #3). The curve shows the final measured ratio as a function of the depletion of undesired cells. For a measured final percentage of 6 % undesired cells after sorting the depletion was 93 %.

c) Purity

As used herein, the purity p is given by the percentage of desired components in the final isolated sample and dependent on the accuracy and depletion as well as the ratio of desired and undesired components in the initial sample:

$$p = \frac{n \text{ desired, isolated}}{n \text{ total, isolated}}.$$

The achieved purity of the individual sorting runs is given in Fig. 16 as the percentage of the respective desired cells after sorting. On average, a purity of $(88 \pm 3)$ % was achieved.

**Example 9 - Image-based identification and separation of W/O Droplets**

An image-based sorting method was performed under similar conditions as described for Example 7, however, with the difference that no encapsulated cells were used. Instead, different patterns were printed into the W/O droplets as described in Example 2, thereby obtaining three visually different kinds of droplets, namely empty droplets (not imprinted), droplets imprinted with a cross, and droplet imprinted with a comet.

Also, image analysis other than the one used for the evaluation of fluorescent droplet-content in Example 7 was used. A convolutional neural network (CNN) was used to differentiate between the different shapes previously printed into W/O droplets containing photoresist. CNN is well known to the skilled person.

CNN Training Data

A convolutional neural network (CNN) was set up for shape detection using the keras framework [5] and according to the architecture shown in Fig. 18b. Fig. 18b shows the neural network architecture used for said image classification. The kernel sizes are denoted in parentheses. In the three convolutional layers 32, 64 and 128 features were extracted, respectively.

The CNN was trained to differentiate between three classes: Droplets containing a cross, a comet, and empty droplets, respectively. In three separate areas, droplets were either left empty, patterned with a cross, or patterned with a comet using a macro used for targeting individual droplets in an area, that was adapted from the selection script. Afterwards, training data was generated with the macro by autonomously imaging about 300 individual droplets within each section. Since all three sections were segregated and contained only droplets of one class, the training data did not have to be manually labelled. Before feeding the acquired images into the network, the images were cropped to only contain the droplet in the center and the resolution of the cropped image was reduced to $50\times50$ pixels, see example images in Fig. 21. To test the required size for this specific set up, smaller subsets of the training data with an equal representation of each class were used to train a neural network, with 10 % of the subset used for validation. The accuracy of the respective neural networks was tested on the entire data set of 900 droplets to confront the network with data it had not seen in training. It showed that the networks would consistently score above 99 % if at least 100 images per class were used for training. For 50 to 100 images per class, the accuracy varied significantly between different training runs. In this range, a score of more than 96 % on the entire data set could still be reliably achieved, if the network was independently trained ten times and out of the ten the best performing network was picked for classification.

## Sorting and Accuracy thereof

The CNN model trained on all available data consistently achieved a score above 99.5 % in training. It was implemented into a new route on the server, which responds with the predicted classification after receiving a file-path to an image acquired by the microscope. The ZEN macro scripts written in the ZEN environment in the language of IronPython 2.7 connected with separate image analysis scripts in CPython 3 environment were adapted to now request the evaluation of the droplet content from this new route. The macro was further adapted to randomly skip or print one of the patterns into droplets within an area of 1 mm$^2$, and to subsequently select one pattern in this area, as seen in Fig. 18c and Fig. 18d. The images of the classified droplets were saved with their predicted label and reviewed.

The accuracy of the model for the real sample was determined to be 96.4 % (N=1314). No cross patterns were misclassified, while 5.2 % of comets and 4.9 % of empty droplets were wrongly identified as another class. The majority (60 %) of wrongly classified droplets were either partly obscured by stacked droplets or their appearance was altered by scratches in the top of the chamber above them, which resembled the cross or comet pattern, leading the model to classify them accordingly.

Fig. 18a shows the three visually different droplets used to train the CNN: Cross, empty and comet (from the top).

Fig. 18b shows the neural network architecture used for image classification. The kernel sizes are denoted in parentheses. In the three convolutional layers 32, 64 and 128 features were extracted, respectively.

Fig. 18c shows the sorting, i.e., identification and polymerization, of droplets containing a cross imprint.

Fig. 18d shows the sorting, i.e., identification and polymerization, of droplets containing a comet imprint.

## Example 10 - **Curing of double emulsions**

As a proof-of-concept, the oil shell of double emulsions (W/O/W droplets) was cured by illumination with a light having a wavelength of 365 nm.

Fig. 18a shows producing of W/O/W droplets in two separated chips. W/O droplets, previously produced in a microfluidic chip with a channel width of 30 μm, were infused into the main channel of another 30 μm microfluidic chip with PVA coating (left). 50 mg/mL PVA solution was connected to the side channels. In this configuration stable W/O/W droplets with a diameter of 23 μm and average shell thickness of 2 μm were produced (right).

Fig. 18b shows producing of W/O/W droplets in two connected chips. In a first 30 μm microfluidic chip with hydrophobic coating W/O droplets were created. By changing the water-oil ratio in the first chip the distance between W/O droplets in the outlet were tuned (left). The outlet of this chip is connected to a second 80 μm microfluidic chip with hydrophilic coating, in which the W/O droplets were encapsulated again to form W/O/W droplets (center-left). Mono-disperse W/O/W droplets with a core size of 28 μm and a shell thickness of 35 μm were created. The inner and outer aqueous phases were dyed with 0.075 wt% Methylene blue (center-right). After 3 days the core had shrunken significantly in size (right).

Fig. 18c shows polymerizing the shell of loosely packed W/O/W droplets: Time series from left to right, with an interval of 2.1 s. The droplet was moving during printing, presumably as the result of the generation of heat during polymerization.

The following summarizes embodiments of the present invention and preferred aspects thereof, it is to be understood that these embodiments and preferred aspects are disclosed in combination with any of the claims and further features described herein:

Embodiments:

1. A method for identifying and separating a specific component of interest from a plurality of components, the method comprises the steps of:

A) providing a dispersion comprising the plurality of components, optionally enclosed in compartments, and a photoresist, wherein among the plurality of components at least one portion shows a factor of interest;

B) scanning of the dispersion for at least one factor of interest to identify said at least one portion among the plurality of components or optionally of the compartments and selecting those components or optionally compartments of interest and attributing a location to the components or optionally compartments of interest within the dispersion;

C) applying a light to at least one part of the dispersion for curing said photoresist either attributed to the components of interest or not attributed to the components of interest; and

D) separating a component from parts of the dispersion with cured photoresist from components from other parts of the dispersion with uncured photoresist.

2. Method according to embodiment 1,
wherein the compartment is one of a droplet, preferably a water-in-oil droplet or a double emulsion droplet, a vesicle, preferably a multilamellar vesicle, a unilamellar vesicle or a GUV, or a coacervate, in which the components are encapsulated.

3. Method according to embodiment 1 or 2,
wherein the component is a natural cell, synthetic cell, organelle, protein, nucleic acid, or inorganic particle.

4. Method according to any one of embodiments 1 to 3,
wherein the component of interest or optionally compartment of interest differs from the other components or optionally from the other compartments by an optical feature, such as a fluorescent moiety and/or the shape and/or behavior of the component.

5. Method according to any one of embodiments 1 to 4,
wherein the dispersion has a continuous phase that is oil-based or water-based.

6. Method according to any one of claims 1 to 5,
wherein the photoresist is a biocompatible photoresist.

7. Method according to any one of embodiments 1 to 6,
wherein the photoresist is a negative photoresist and includes a monomer component and an initiator component.

8. Method according to any one of embodiments 1 to 7,
wherein the light is an LED or a laser, in particular wherein the light is a focused light.

9. Method according to any one of claims 1 to 8,
wherein in step C) the light is configured to selectively illuminate the at least one part of the dispersion.

10. Method according to any one of embodiments 1 to 9,
wherein in step A) the dispersion is provided in a chamber having an array of sub-chambers, each being designed to take up one or more components.

11. Method according to embodiment 10,

wherein the chamber is designed to have only one layer of components, optionally enclosed in compartments, and/or
wherein the concentration of components, optionally enclosed in compartments, in step A) is adjusted so that a single layer of components, optionally enclosed in compartments, is formed within the chamber.

12. Method according to embodiment 10 or 11, wherein
the method further comprises step B0) prior to step B), wherein B0) is scanning of the chamber to determine a suitable depth of focus for a camera observing the chamber.

13. Method according to any one of embodiments 1 to 12,
wherein the method is carried out by utilizing a microscope having a light source suitable for polymerizing the photoresist.

14. Method according to any one of embodiments 1 to 13,
wherein the method is carried out by utilizing a microscope having a camera, and wherein in step B) an image is taken by the camera and analyzed, in particular by a computer, for at least one factor of interest.

15. Method according to any one of embodiments 1 to 14,
wherein the method is carried out by utilizing a microscope providing structured illumination, preferably wherein the microscope has a digital micromirror device, to allow curing of the photoresist in step C) at several positions in parallel.

16. Method according to any one of embodiments 1 to 15,

wherein the method is in part a computer-implemented method, in particular wherein the computer-implemented method includes in optional step B0) the scanning of the chamber is configured to determine a depth of focus for taking images of the chamber,

in step B) the acquisition of an image and analysis of the image for at least one factor of interest and attributing a location to the components of interest within the dispersion, and

in step C) controlling a light source to selectively apply light to the photoresist at a portion desired to cure said photoresist.

17. Method according to any one of embodiments 1 to 16,

wherein step B) and/or step C) is carried out automatically, in particular based on machine learning driven image analysis.

18. Method according to any one of embodiments 1 to 17,

wherein in step B) the image acquisition is controlled by the computer analyzing the image, adjusting the focus and/or coordinates of the image taken.

19. Method according to any one of embodiments 1 to 18,

wherein step B) and step C) are repeated multiple times.

20. Method according to any one of embodiments 1 to 19,

wherein in step A) components are provided in an amount of at least $10^2$ cells per total volume of the dispersion, preferably in an amount lying in a range of from $10^5$ to $10^7$ cells per total volume of the dispersion.

21. Method according to any one of embodiments 1 to 20,

wherein the components are enclosed in compartments, and wherein the compartments are essentially round and have a diameter in the range of 1 $\mu$m to 10.000 $\mu$m, more preferably 3 $\mu$m to 1000 $\mu$m, even more preferably 5 $\mu$m to 500 $\mu$m, most preferably 10 $\mu$m to 200 $\mu$m.

22. Device configured to carry out the method according to any one of embodiments 1 to 21, preferably wherein the device comprises a microscope.

Reference signs

1    Light source
1a   Illumination

2    Objective
3    Polymerized/cured photoresist
4    Component (of interest / not of interest)
5    Chamber
6    Continuous phase
7    W/O droplet
8    Selection chamber
9    Double emulsion (W/O/W droplet)
10   Oil Shell
11   Inner aqueous phase
12   Microplate
13   Glass slide
14   PDMS chip
15   DMD
16   Stage

References

[1] Cody A. LaBelle, Angelo Massaro, Belén Cortés-Llanos, Christopher E. Sims, and Nancy L. Allbritton. Image-based live cell sorting. Trends in Biotechnology, 39(6):613-623, 2021.

[2] C. Bradford Barber, David P. Dobkin, and Hannu Huhdanpaa. The quickhull algorithm for convex hulls. ACM Trans. Math. Softw., 22(4):469-483, dec 1996.

[3] G. Bradski. The OpenCV Library. Dr. Dobb's Journal of Software Tools, 2000.

[4] Johannes Schindelin, Ignacio Arganda-Carreras, Erwin Frise, Verena Kaynig, Mark Longair, Tobias Pietzsch, Stephan Preibisch, Curtis Rueden, Stephan Saal-feld, Benjamin Schmid, Jean-Yves Tinevez, Daniel James White, Volker Hartenstein, Kevin Eliceiri, Pavel Tomancak, and Albert Cardona. Fiji: an open-source platform for biological-image analysis. Nat. Methods, 9(7):676-682, June 2012.

[5] François Chollet et al. Keras. https://keras.io, 2015.

The content of all references is incorporated by reference in their entirety.

**Claims**

1. A method for identifying and separating a specific component of interest from a plurality of components, the method comprises the steps of:

    A) providing a dispersion comprising the plurality of components, optionally enclosed in compartments, and a photoresist, wherein among the plurality of components at least one portion shows a factor of interest;

B) scanning of the dispersion for at least one factor of interest to identify said at least one portion among the plurality of components or optionally of the compartments and selecting those components or optionally compartments of interest and attributing a location to the components or optionally compartments of interest within the dispersion;

C) applying a light to at least one part of the dispersion for curing said photoresist either attributed to the components of interest or not attributed to the components of interest; and

D) separating a component from parts of the dispersion with cured photoresist from components from other parts of the dispersion with uncured photoresist.

2. Method according to claim 1,
   wherein the compartment is one of a droplet, preferably a water-in-oil droplet or a double emulsion droplet, a vesicle, preferably a multilamellar vesicle, a unilamellar vesicle or a GUV, or a coacervate, in which the components are encapsulated.

3. Method according to claim 1 or 2,
   wherein the component is a natural cell, synthetic cell, organelle, protein, nucleic acid, or inorganic particle.

4. Method according to any one of claims 1 to 3,
   wherein the component of interest or optionally compartment of interest differs from the other components or optionally from the other compartments by an optical feature, such as a fluorescent moiety and/or the shape and/or behavior of the component.

5. Method according to any one of claims 1 to 4,
   wherein the photoresist is a negative photoresist and includes a monomer component and an initiator component.

6. Method according to any one of claims 1 to 5,
   wherein the photoresist is a biocompatible photoresist.

7. Method according to any one of claims 1 to 6,
   wherein the light is an LED or a laser beam, in particular wherein the light is a focused light.

8. Method according to any one of claims 1 to 7,
   wherein in step C) the light is configured to selectively illuminate the at least one part of the dispersion.

9. Method according to any one of claims 1 to 8,

wherein in step A) the dispersion is provided in a chamber having an array of sub-chambers, each being designed to take up one or more components, preferably wherein the chamber is designed to have only one layer of components, optionally enclosed in compartments, and/or wherein the concentration of components, optionally enclosed in compartments, in step A) is adjusted so that a single layer of components, optionally enclosed in compartments, is formed within the chamber.

10. Method according to claim 9, wherein
    the method further comprises step B0) prior to step B), wherein B0) is scanning of the chamber to determine a suitable depth of focus for a camera observing the chamber.

11. Method according to any one of claims 1 to 10,

    wherein the method is carried out by utilizing a microscope having a light source suitable for polymerizing the photoresist; and/or
    wherein the method is carried out by utilizing a microscope having a camera, and wherein in step B) an image is taken by the camera and analyzed, in particular by a computer, for at least one factor of interest.

12. Method according to any one of claims 1 to 11,
    wherein the method is carried out by utilizing a microscope providing structured illumination, preferably wherein the microscope has a digital micromirror device, to allow curing of the photoresist in step C) at several positions in parallel.

13. Method according to any one of claims 1 to 12,

    wherein the method is in part a computer-implemented method, in particular wherein the computer-implemented method includes in optional step B0) the scanning of the chamber is configured to determine a depth of focus for taking images of the chamber,
    in step B) the acquisition of an image and analysis of the image for at least one factor of interest and attributing a location to the components of interest within the dispersion, and
    in step C) controlling a light source to selectively apply light to the photoresist at a portion desired to cure said photoresist.

14. Method according to any one of claims 1 to 13,

    wherein step B) and/or step C) is carried out automatically, in particular based on machine learning driven image analysis; and/or
    wherein in step B) the image acquisition is controlled by the computer analyzing the image, adjusting the focus and/or coordinates of the image taken; and/or

wherein step B) and step C) are repeated multiple times.

15. Device configured to carry out the method according to any one of claims 1 to 14, preferably wherein the device comprises a microscope.

Fig.1

*Fig.2*

Top View:

7

8

3

4

6

*Fig.3*

Top View:

Fig.4

Fig.5a-b

*Fig.6a-c*

Fig.7a-c

Fig.8a-c

EP 4 478 027 A1

Fig.9a-b

a

7

6

12

2

b

13

14

c

*Fig.10a-c*

Fig.11a

⑤

⑥

⑦

*Fig.11b*

Fig.11c

⑬

⑭

⑮

*Fig.11d*

Fig.12

User sets corner points of target area to be searched

wait for user input

Stage moves to different positions equally distributed within target area

User sets focus at each position

Determine droplet plane by interpolation of entered focused positions (/setfocus)

Divide selected target area into tiles & move to top-left tile

Adjust focus according to droplet plane (/getfocus)

Acquire image and detect all droplets within field of view (/detectdroplets)

[Select **GFP/Cy3** Reflector]

Analyze content of each detected droplet individually (/scancircle, /detectpattern)

[Select **DAPI** Reflector]

Move to next tile

Polymerize photoresist in droplets selected for elimination

*Fig.13*

Fig.14a-d

Fig.15

Fig.16

Fig.17

Fig.18a-d

*Fig.19a-d*

*Fig.20*

*Fig.21*

Europäisches
Patentamt

European
Patent Office

Office européen
des brevets

# EUROPEAN SEARCH REPORT

Application Number

EP 23 17 9532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2021/172856 A1 (ZHAO QI [US]) 10 June 2021 (2021-06-10) * paragraphs [0003], [0005] – [0011], [0018], [0025] – [0033], [0042], [0043], [0053] – [0060], [0093], [0124] – [0126]; figure 1 * * paragraphs [0130] – [0137], [0144], [0150], [0160], [0179] * | 1–15 | INV. G01N15/14 ADD. G01N15/10 |
| X | US 2021/403942 A1 (WANG STAN [US] ET AL) 30 December 2021 (2021-12-30) * paragraphs [0007], [0015], [0016], [0018], [0020], [0025], [0342], [0345], [0356], [0371] – [0373]; figures 20,21,27,30,35-43; tables 1-3 * * paragraphs [0389], [0408], [0421], [0425], [0465], [0519] * | 1,3–15 | |
| X | EP 3 214 166 A1 (UNIV TOKYO NAT UNIV CORP [JP]; HITACHI CHEMICAL CO LTD [JP]) 6 September 2017 (2017-09-06) * paragraphs [0017], [0035], [0040]; figure 5 * | 1,3, 7–12,15 | |

TECHNICAL FIELDS
SEARCHED      (IPC)

G01N

The present search report has been drawn up for all claims

| Place of search | Date of completion of the search | Examiner |
|---|---|---|
| The Hague | 10 November 2023 | Geromel Prette, A |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 23 17 9532

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2023

| Patent document cited in search report | Publication date | Patent family member(s) | | Publication date |
|---|---|---|---|---|
| US 2021172856 A1 | 10-06-2021 | CN | 112867916 A | 28-05-2021 |
| | | EP | 3830546 A1 | 09-06-2021 |
| | | JP | 7371098 B2 | 30-10-2023 |
| | | JP | 2021533830 A | 09-12-2021 |
| | | JP | 2023095990 A | 06-07-2023 |
| | | US | 2021172856 A1 | 10-06-2021 |
| | | WO | 2020037176 A1 | 20-02-2020 |
| US 2021403942 A1 | 30-12-2021 | EP | 3877748 A1 | 15-09-2021 |
| | | US | 2021403942 A1 | 30-12-2021 |
| | | WO | 2020097083 A1 | 14-05-2020 |
| EP 3214166 A1 | 06-09-2017 | CN | 107075447 A | 18-08-2017 |
| | | EP | 3214166 A1 | 06-09-2017 |
| | | JP | 6619353 B2 | 11-12-2019 |
| | | JP | WO2016068249 A1 | 10-08-2017 |
| | | KR | 20170074237 A | 29-06-2017 |
| | | SG | 11201703487T A | 29-06-2017 |
| | | TW | 201627501 A | 01-08-2016 |
| | | US | 2017321183 A1 | 09-11-2017 |
| | | WO | 2016068249 A1 | 06-05-2016 |

EPO FORM P0459

For more details about this annex : see Official Journal of the European Patent Office, No. 12/82

**REFERENCES CITED IN THE DESCRIPTION**

**Non-patent literature cited in the description**

- **JI-WON KIM** ; **SANG HOON HAN** ; **YE HUN CHOI** ; **WAHYU MARTUMPAL HAMONANGAN** ; **YOON-JIN OH** ; **SHIN-HYUN KIM**. Recent advances in the microfluidic production of functional microcapsules by multiple-emulsion templating. *Lab Chip*, 2022, vol. 22, 2259-2291

- **CODY A. LABELLE** ; **ANGELO MASSARO** ; **BELÉN CORTÉS-LLANOS** ; **CHRISTOPHER E. SIMS** ; **NANCY L. ALLBRITTON**. Image-based live cell sorting. *Trends in Biotechnology*, 2021, vol. 39 (6), 613-623

- **C. BRADFORD BARBER** ; **DAVID P. DOBKIN** ; **HANNU HUHDANPAA**. The quickhull algorithm for convex hulls. *ACM Trans. Math. Softw.*, December 1996, vol. 22 (4), 469-483

- **G. BRADSKI**. The OpenCV Library. *Dr. Dobb's Journal of Software Tools*, 2000

- **JOHANNES SCHINDELIN** ; **IGNACIO ARGANDA-CARRERAS** ; **ERWIN FRISE** ; **VERENA KAYNIG** ; **MARK LONGAIR** ; **TOBIAS PIETZSCH** ; **STEPHAN PREIBISCH** ; **CURTIS RUEDEN** ; **STEPHAN SAAL-FELD** ; **BENJAMIN SCHMID**. Fiji: an open-source platform for biological-image analysis. *Nat. Methods*, June 2012, vol. 9 (7), 676-682

- **FRANÇOIS CHOLLET et al.** *Keras*, 2015, https://keras.io